(19) Europäisches Patentamt
European Patent Office
Office européen des brevets

(11) EP 1 174 405 B1

(12) **EUROPÄISCHE PATENTSCHRIFT**

(45) Veröffentlichungstag und Bekanntmachung des Hinweises auf die Patenterteilung:
**25.01.2006 Patentblatt 2006/04**

(51) Int Cl.:
*C07C 13/28* (2006.01) *C09K 19/30* (2006.01)
*C07C 22/00* (2006.01) *C07D 319/06* (2006.01)
*C09K 19/34* (2006.01)

(21) Anmeldenummer: **01115391.3**

(22) Anmeldetag: **25.06.2001**

(54) **Dialkinverbindungen**

Dialkyne compounds

Composés de dialcyne

(84) Benannte Vertragsstaaten:
**DE GB**

(30) Priorität: **20.07.2000 DE 10035651**

(43) Veröffentlichungstag der Anmeldung:
**23.01.2002 Patentblatt 2002/04**

(73) Patentinhaber: **Merck Patent GmbH
64293 Darmstadt (DE)**

(72) Erfinder:
- **Reiffenrath, Volker
64380 Rossdorf (DE)**
- **Lüssem, Georg, Dr.
64372 Ober-Ramstadt (DE)**

(56) Entgegenhaltungen:
**EP-A- 1 006 098** **DE-A- 19 959 723**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**EP 1 174 405 B1**

**Beschreibung**

Die Erfindung betrifft Dialkinverbindungen der Formel I

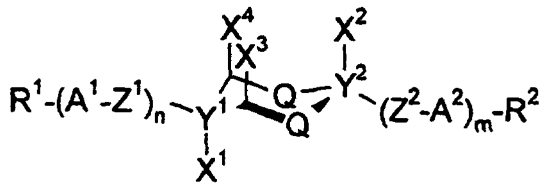

I

worin

| | |
|---|---|
| $R^1$, $R^2$ | unabhängig voneinander H, F, einen unsubstituierten, einen mindestens einfach durch Halogen oder CN substituierten Alkylrest mit 1-15 C-Atomen, worin auch eine oder mehrere $CH_2$-Gruppen jeweils unabhängig voneinander durch -O-, -S-, -CO-, 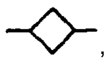, -CO-O-, -O-CO-, -O-CO-O- oder -CH=CH- so ersetzt sein können, dass Heteroatome nicht direkt verbunden sind. |
| $X^1$ , $X^2$, $X^3$, $X^4$ | jeweils unabhängig voneinander H oder -C≡C-C≡C-$R^3$, wobei zumindest eine der Gruppen $X_1$, $X_2$, $X_3$ und $X^4$ -C≡C-C≡C-$R^3$ bedeutet, |
| $R^3$ | H, Cl, CN, $SF_5$, $CF_3$, einen unsubstituierten oder einen mindestens einfach durch Halogen substituierten Alkylrest mit 1-15 C-Atomen, worin auch eine oder mehrere $CH_2$-Gruppen durch jeweils unabhängig voneinander -CH=CH- oder -O- so ersetzt sein können, dass -O-Atome nicht direkt verbunden sind, |
| Q | -$CH_2$- oder -O-, |

| | |
|---|---|
| und $Y^1$, $Y^2$ | unabhängig voneinander C oder Si, |
| $A^1$, $A^2$ | unabhängig voneinander einen unsubstituierten oder durch F oder CN substituierten trans-1,4-Cyclohexylenrest, worin auch eine oder mehrere nicht benachbarte $CH_2$-Gruppen durch -O- und/oder -S- ersetzt sein können oder |

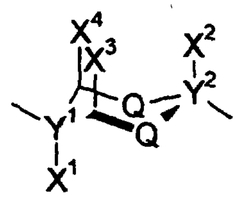

,

| | |
|---|---|
| $Z^1$ , $Z^2$ | jeweils unabhängig voneinander -CO-O-, -O-CO-, -$CH_2$O--O-, -O$CH_2$-, -$CF_2$O-, -O$CF_2$-, -$CF_2CF_2$-, -CF=CF-, -$CH_2CH_2$-, -CH=CH- oder eine Einfachbindung und |
| n, m | unabhängig voneinander 0, 1, 2 oder 3, wobei |
| m+n | 1,2 oder 3. |

bedeutet.

Die Erfindung betrifft außerdem die Verwendung der Verbindungen der Formel I als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I weisen häufig einen geringen positiven oder negativen Wert der dielektrischen Anisotropie auf und können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (Electrically controlled birefringence) oder dem Effekt der dynamischen Streuung beruhen.

Die bisher für diesen Zweck eingesetzten Substanzen haben stets gewisse Nachteile, beispielsweise zu geringe Stabilität gegenüber der Einwirkung von Wärme, Licht oder elektrischen Feldern, ungünstige elastische und/oder dielektrische Eigenschaften.

Der Erfindung lag die Aufgabe zugrunde, neue stabile flüssigkristalline oder mesogene Verbindungen mit besonders kleiner optischer Anisotropie ($\Delta n$) und negativer oder positiver dielektrischen Anisotropie ($\Delta\varepsilon$) aufzufinden, die als Komponenten flüssigkristalliner Medien, insbesondere für TFT- und STN-Displays, geeignet sind.

Es wurde nun gefunden, dass die Verbindungen der Formel I vorzüglich als Komponenten flüssigkristalliner Medien geeignet sind. Mit ihrer Hilfe lassen sich stabile flüssigkristalline Medien, insbesondere geeignet für TFT- oder STN-Displays, erhalten. Die neuen Verbindungen zeichnen sich vor allem durch eine hohe thermische Stabilität aus, die für eine hohe "holding ratio" vorteilhaft ist und zeigen günstige Werte der Klärpunkte. Die Verbindungen der Formel I weisen bei einer reduzierten Temperatur von 0,9 und einer Wellenlänge von 589 nm einen Wert für die optische Anisotropie $\Delta n$ von < 0,03, vorzugsweise von < 0,02 auf, der auf einen besonders großen Wert von $n_{\perp}$ zurückgeht. Hierbei ist die reduzierte Temperatur wie folgt definiert:

$$\frac{\text{Meßtemperatur in K}}{\text{Klärpunktstemperatur in K}} = \text{reduzierte Temperatur}$$

Flüssigkristalline Medien mit sehr kleinen Werten der optischen Anisotropie sind insbesondere für reflektive und transflektive Anwendungen von Bedeutung, d.h. solche Anwendungen, bei denen das jeweilige LCD keine oder nur unterstützende Hintergrundbeleuchtung erfährt. Geringe Werte des $\Delta n$ werden durch Verwendung von Substituenten $X^1$, $X^2$, $X^3$ und/oder $X^4$ mit möglichst hoher Polarisierbarkeit erreicht. Aufgrund des kleinen Volumens der Gruppen $X^1$, $X^2$, $X^3$ und $X^4$ werden die übrigen LC-Eigenschaften, wie Klärpunkt und Viskosität, von flüssigkristallinen Mischungen, die mit den erfindungsgemäßen Verbindungen versetzt werden, nur relativ gering beeinträchtigt.

Mit der Bereitstellung von Verbindungen der Formel I wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren. Durch Zusatz der Verbindungen der Formel I zu flüssigkristallinen Dielektrika lassen sich $\Delta n$-Werte solcher Medien deutlich reduzieren.

Die Bedeutung der Formel I schließt alle Isotope der in den Verbindungen der Formel I gebundenen chemischen Elemente ein. In enantiomerenreiner oder -angereicherter Form eignen sich die Verbindungen der Formel I auch als chirale Dotierstoffe und generell zur Erzielung chiraler Mesophasen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektrooptische Anzeigeelemente, die derartige Medien enthalten.

Vor- und nachstehend haben n, m, $R^1$, $R^2$, $R^3$, $X^1$, $X^2$, $X^3$, $X^4$, $Z^1$, $Z^2$, $A^1$ $A^2$, Q, $Y^1$ und $Y^2$ die angegebene Bedeutung, sofern nicht ausdrücklich etwas anderes vermerkt ist. Kommt der Rest $X^1$ mehrfach vor, so kann er gleiche oder verschiedene Bedeutungen annehmen. Dasselbe gilt für alle anderen mehrfach auftretenden Gruppen.

Der Einfachheit halber bedeuten im folgenden Cyc einen Cyclohexan-1,4-diylrest oder einen 1- oder 4-Silacyclohexan-1,4-diylrest, Dio einen 1,3-Dioxan-2,5-diylrest, Dit einen 1,3-Dithian-2,5-diylrest, einen Bicyclo-(2,2,2)-octylenrest, wobei Cyc unsubstituiert oder ein- oder mehrfach durch F oder CN substituiert sein kann.

W bedeutet das folgende Strukturelement:

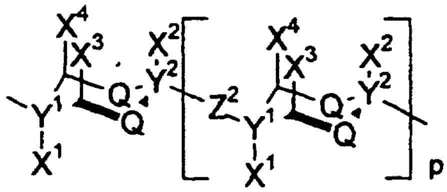

worin $X^1$, $X^2$, $X^3$, $X^4$, Q, $Y^1$, $Y^2$ und $Z^2$ die oben angegebene Bedeutung aufweisen und p 0, 1, 2 oder 3 bedeutet.

Bevorzugte Bedeutungen der Gruppe W geben die Teilformeln W1 bis W7 wieder:

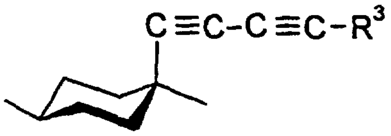

W1

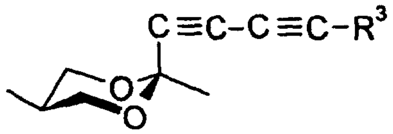

W2

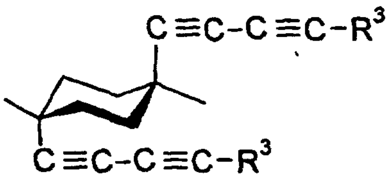

W3

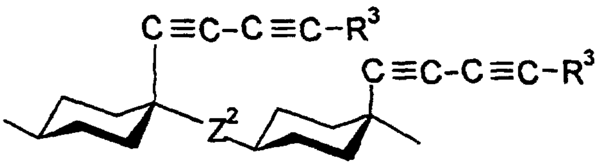

W4

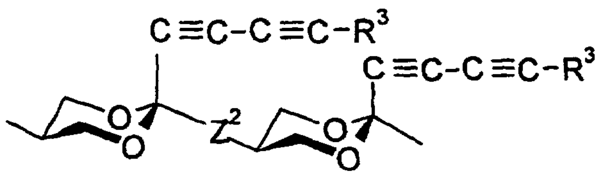

W5

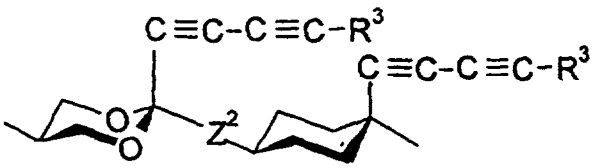

W6

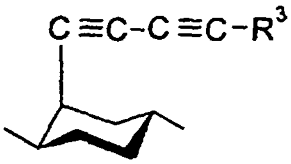

W7

worin $Z^2$ und $R^3$ die oben angegebenen Bedeutungen aufweisen.

Formel I umfaßt die bevorzugten Verbindungen der Teilformeln Ia1 bis Ia12, die neben der Gruppe W einen sechsgliedrigen Ring enthalten:

$R^1$-W-Cyc-$R^2$ Ia1

$R^1$-W-$CH_2CH_2$-Cyc-$R^2$ Ia2

$R^1$-W-COO-Cyc-$R^2$ Ia3

$R^1$-W-Dio-$R^2$ Ia4

$R^1$-W-$CH_2CH_2$-Dio-$R^2$ Ia5

$R^1$-W-COO-Dio-$R^2$ Ia6

$R^1$-Cyc-W-$R^2$ Ia7

$R^1$-Dio-W-$R^2$ Ia8

$R^1$-Cyc-$CH_2CH_2$-W-$R^2$ Ia9

$R^1$-Dio-$CH_2CH_2$-W-$R^2$ Ia10

$R^1$-Cyc-COO-W-$R^2$ Ia11

$R^1$-Dio-COO-W-$R^2$ Ia12

ferner die ebenfalls bevorzugten Verbindungen der Teilformeln Ib1 bis Ib72, die zusätzlich zur Gruppe W zwei sechsgliedrige Ringe enthalten:

$R^1$-Cyc-Cyc-W-$R^2$ Ib1

$R^1$-Dio-Cyc-W-$R^2$ Ib2

$R^1$-Cyc-$CH_2CH_2$-Cyc-W-$R^2$ Ib3

$R^1$-Dio-$CH_2CH_2$-Cyc-W-$R^2$ Ib4

$R^1$-Cyc-COO-Cyc-W-$R^2$ Ib5

$R^1$-Dio-COO-Cyc-W-$R^2$ Ib6

$R^1$-Cyc-Dio-W-$R^2$ Ib7

$R^1$-Dio-Dio-W-$R^2$ Ib8

$R^1$-Cyc-$CH_2CH_2$-Dio-W-$R^2$ Ib9

$R^1$-Dio-$CH_2CH_2$-Dio-W-$R^2$ Ib10

$R^1$-Cyc-COO-Dio-W-$R^2$ Ib11

$R^1$-Dio-COO-Dio-W-$R^2$ Ib12

$R^1$-Cyc-Cyc-$CH_2CH_2$-W-$R^2$ Ib13

$R^1$-Dio-Cyc-$CH_2CH_2$-W-$R^2$ Ib14

$R^1$-Cyc-Dio-$CH_2CH_2$-W-$R^2$ Ib15

$R^1$-Dio-Dio-$CH_2CH_2$-W-$R^2$ Ib16

$R^1$-Cyc-Cyc-COO-W-$R^2$ Ib17

$R^1$-Dio-Cyc-COO-W-$R^2$ Ib18

$R^1$-Cyc-Dio-COO-W-$R^2$ Ib19

$R^1$-Dio-Dio-COO-W-$R^2$ Ib20

$R^1$-Cyc-W-Cyc-$R^2$ Ib21

$R^1$-Dio-W-Cyc-$R^2$ Ib22

$R^1$-Cyc-$CH_2CH_2$-W-Cyc-$R^2$ Ib23

$R^1$-Dio-$CH_2CH_2$-W-Cyc-$R^2$ Ib24

$R^1$-Cyc-COO-W-Cyc-$R^2$ Ib25

$R^1$-Dio-COO-W-Cyc-$R^2$ Ib26

$R^1$-Cyc-W-$CH_2CH_2$-Cyc-$R^2$ Ib27

$R^1$-Dio-W-$CH_2CH_2$-Cyc-$R^2$ Ib28

$R^1$-Cyc-W-COO-Cyc-$R^2$ Ib29

$R^1$-Dio-W-COO-Cyc-$R^2$ Ib30

$R^1$-Cyc-W-Dio-$R^2$ Ib31

$R^1$-Dio-W-Dio-$R^2$ Ib32

$R^1$-Cyc-$CH_2CH_2$-W-Dio-$R^2$ Ib33

$R^1$-Dio-$CH_2CH_2$-W-Dio-$R^2$ Ib34

$R^1$-Cyc-COO-W-Dio-$R^2$ Ib35

$R^1$-Dio-COO-W-Dio-$R^2$ Ib36

$R^1$-Cyc-W-$CH_2CH_2$-Dio-$R^2$ Ib37

$R^1$-Dio-W-$CH_2CH_2$-Dio-$R^2$ Ib38

$R^1$-Cyc-W-COO-Dio-$R^2$ Ib39

$R^1$-Dio-W-COO-Dio-$R^2$ Ib40

$R^1$-W-Cyc-Cyc-$R^2$ Ib41

$R^1$-W-$CH_2CH_2$-Cyc-Cyc-$R^2$ Ib42

$R^1$-W-COO-Cyc-Cyc-$R^2$ Ib43

$R^1$-W-Dio-Cyc-$R^2$ Ib44

$R^1$-W-$CH_2CH_2$-Dio-Cyc-$R^2$ Ib45

$R^1$-W-COO-Dio-Cyc-$R^2$ Ib46

$R^1$-W-Cyc-$CH_2CH_2$-Cyc-$R^2$ Ib47

$R^1$-W-Dio-$CH_2CH_2$-Cyc-$R^2$ Ib48

$R^1$-W-Cyc-COO-Cyc-$R^2$ Ib49

$R^1$-W-Dio-COO-Cyc-$R^2$ Ib50

$R^1$-W-Cyc-Dio-$R^2$ Ib51

$R^1$-W-$CH_2CH_2$-Cyc-Dio-$R^2$ Ib52

$R^1$-W-COO-Cyc-Dio-$R^2$ Ib53

$R^1$-W-Dio-Dio-$R^2$ Ib54

$R^1$-W-$CH_2CH_2$-Dio-Dio-$R^2$ Ib55

$R^1$-W-COO-Dio-Dio-$R^2$ Ib56

$R^1$-W-Cyc-$CH_2CH_2$-Dio-$R^2$ Ib57

$R^1$-W-Dio-$CH_2CH_2$-Dio-$R^2$ Ib58

$R^1$-W-Cyc-COO-Dio-$R^2$ Ib59

$R^1$-W-Dio-COO-Dio-$R^2$ Ib60

$R^1$-Cyc-$CH_2CH_2$-W-$CH_2CH_2$-Cyc-$R^2$ Ib61

$R^1$-Dio-$CH_2CH_2$-W-$CH_2CH_2$-Cyc-$R^2$ Ib62

$R^1$-Cyc-$CH_2CH_2$-W-$CH_2CH_2$-Dio-$R^2$ Ib63

$R^1$-Dio-$CH_2CH_2$-W-$CH_2CH_2$-Dio-$R^2$ Ib64

$R^1$-Cyc-$CH_2CH_2$-Cyc-$CH_2CH_2$-W-$R^2$ Ib65

$R^1$-Dio-$CH_2CH_2$Cyc-$CH_2CH_2$-W-$R^2$ Ib66

$R^1$-Cyc-$CH_2CH_2$-Dio-$CH_2CH_2$-W-$R^2$ Ib67

$R^1$-Dio-$CH_2CH_2$-Dio-$CH_2CH_2$-W-$R^2$ Ib68

$R^1$-W-$CH_2CH_2$-Cyc-$CH_2CH_2$-Cyc-$R^2$ Ib69

$R^1$-W-$CH_2CH_2$-Dio-$CH_2CH_2$-Cyc-$R^2$ Ib70

$R^1$-W-$CH_2CH_2$-Cyc-$CH_2CH_2$Dio-$R^2$ Ib71

$R^1$-W-$CH_2CH_2$-Dio-$CH_2CH_2$-Dio-$R^2$ Ib72

sowie die bevorzugten Verbindungen der Teilformeln Ic1 bis Ic55, die neben der Gruppe W drei sechsgliedrige Ringe enthalten:

$R^1$-W-Cyc-Cyc-Cyc-$R^2$ Ic1

$R^1$-W-$CH_2CH_2$-Cyc-Cyc-Cyc-$R^2$ Ic2

$R^1$-W-Dio-Cyc-Cyc-$R^2$ Ic3

$R^1$-W-$CH_2CH_2$-Dio-Cyc-Cyc-$R^2$ Ic4

$R^1$-W-Cyc-$CH_2CH_2$-Cyc-Cyc-$R^2$ Ic5

$R^1$-W-Dio-$CH_2CH_2$-Cyc-Cyc-$R^2$ Ic6

$R^1$-W-Cyc-Cyc-$CH_2CH_2$-Cyc-$R^2$ Ic7

$R^1$-W-Dio-Cyc-$CH_2CH_2$-Cyc-$R^2$ Ic8

$R^1$-W-Cyc-Dio-Cyc-$R^2$ Ic9

$R^1$-W-$CH_2CH_2$-Cyc-Dio-Cyc-$R^2$ Ic10

$R^1$-W-Dio-Dio-Cyc-$R^2$ Ic11

$R^1$-W-$CH_2CH_2$-Dio-Dio-Cyc-$R^2$ Ic12

$R^1$-W-Cyc-$CH_2CH_2$-Dio-Cyc-$R^2$ Ic13

$R^1$-W-Dio-$CH_2CH_2$-Dio-Cyc-$R^2$ Ic14

$R^1$-W-Cyc-Dio-$CH_2CH_2$-Cyc-$R^2$ Ic15

$R^1$-Cyc-Dio-$CH_2CH_2$-Cyc-W-$R^2$ Ic16

$R^1$-Dio-Dio-$CH_2CH_2$-Cyc-W-$R^2$ Ic17

$R^1$-Cyc-Cyc-Cyc-$CH_2CH_2$-W-$R^2$ Ic18

$R^1$-Dio-Cyc-Cyc-$CH_2CH_2$-W-$R^2$ Ic19

$R^1$-Cyc-Dio-Cyc-$CH_2CH_2$W-$R^2$ Ic20

$R^1$-Dio-Dio-Cyc-$CH_2CH_2$-W-$R^2$ Ic21

$R^1$-Cyc-Cyc-Dio-W-$R^2$ Ic22

$R^1$-Dio-Cyc-Dio-W-$R^2$ Ic23

$R^1$-Cyc-$CH_2CH_2$-Cyc-Dio-W-$R^2$ Ic24

$R^1$-Dio-$CH_2CH_2$-Cyc-Dio-W-$R^2$ Ic25

$R^1$-Cyc-Dio-Dio-W-$R^2$ Ic26

$R^1$-Dio-Dio-Dio-W-$R^2$ Ic27

$R^1$-Cyc-$CH_2CH_2$-Dio-Dio-W-$R^2$ Ic28

$R^1$-Dio-$CH_2CH_2$-Dio-Dio-W-$R^2$ Ic29

$R^1$-Cyc-Cyc-$CH_2CH_2$-Dio-W-$R^2$ Ic30

$R^1$-Dio-Cyc-$CH_2CH_2$-Dio-W-$R^2$ Ic31

$R^1$-Cyc-$CH_2CH_2$-Dio-W-Dio-$R^2$ Ic32

$R^1$-Dio-$CH_2CH_2$-Dio-W Dio-$R^2$ Ic33

$R^1$-Cyc-Cyc-$CH_2CH_2$-W-Dio-$R^2$ Ic34

$R^1$-Dio-Cyc-$CH_2CH_2$-W-Dio-$R^2$ Ic35

$R^1$-Cyc-Dio-$CH_2CH_2$-W-Dio-$R^2$ Ic36

$R^1$-Dio-Dio-$CH_2CH_2$-W-Dio-$R^2$ Ic37

$R^1$-Cyc-Cyc-W-$CH_2CH_2$Dio-$R^2$ Ic38

$R^1$-Dio-Cyc-W-$CH_2CH_2$-Dio-$R^2$ Ic39

$R^1$-Cyc-Dio-W-$CH_2CH_2$-Dio-$R^2$ Ic40

$R^1$-Dio-Dio-W-$CH_2CH_2$-Dio-$R^2$ Ic41

$R^1$-Cyc-W-Dio-$CH_2CH_2$-Cyc-$R^2$ Ic42

$R^1$-Dio-W-Dio-$CH_2CH_2$-Cyc-$R^2$ Ic43

$R^1$-Cyc-W-Cyc-Dio-$R^2$ Ic44

$R^1$-Dio-W-Cyc-Dio-$R^2$ Ic45

$R^1$-Cyc-$CH_2CH_2$-W-Cyc-Dio-$R^2$ Ic46

$R^1$-Dio-$CH_2CH_2$-W-Cyc-Dio-$R^2$ Ic47

$R^1$-Cyc-W-$CH_2CH_2$-Cyc-Dio-$R^2$ Ic48

$R^1$-Dio-W-$CH_2CH_2$Cyc-Dio-$R^2$ Ic49

$R^1$-Cyc-W-Cyc-$CH_2CH_2$-Dio-$R^2$ Ic50

$R^1$-Dio-W-Cyc-$CH_2CH_2$-Dio-$R^2$ Ic51

$R^1$-Cyc-W-Dio-Dio-$R^2$ Ic52

$R^1$-Dio-W-Dio-Dio-$R^2$ Ic53

$R^1$-Cyc-$CH_2CH_2$-W-Dio-Dio-$R^2$ Ic54

$R^1$-DiO-$CH_2CH_2$-W-Dio-Dio-$R^2$ Ic55

worin $R^1$, $R^2$, Cyc, Dio und W die oben angegebene Bedeutung aufweisen.

Verbindungen der Formel I sind bevorzugt, die keine isolierten oder aromatischen C,C-Doppelbindungen aufweisen.

$R^1$ und $R^2$ bedeuten bevorzugt unabhängig voneinander F, $OCF_3$, $CF_3$, geradkettiges Alkyl oder Alkoxy mit 1 bis 15 C-Atomen, insbesondere Alkyl, Alkenyl, Alkenyloxy oder Alkoxy mit bis zu 7 C-Atomen. Insbesondere bedeutet vorzugsweise nur einer der Reste $R^1$ und $R^2$ einen geradkettigen Alkenyl-, Alkoxy-, Alkenyl- oder Alkenyloxyrest mit bis zu 7 C-Atomen.

In bevorzugten Verbindungen der Formel I nimmt $X^1$, $X^2$, $X^3$ und/oder $X^4$ die Bedeutung -C≡C-C≡C-H, -C≡C-C≡C-Alkyl, -C≡C-C≡C-Cl oder -C≡C-C≡C-CN an, wobei Alkyl ein Alkylrest mit 1 bis 15 C-Atomen bedeutet. Insbesondere ist der Alkylrest verzweigt und bedeutet vorzugsweise tert. Butyl.

In besonders bevorzugten Verbindungen der Formel I bedeuten $X^3$ und $X^4$ gleichzeitig H.

Weiterhin sind solche Verbindungen der Formel I bevorzugt, in denen nur eine der Gruppen $X^1$, $X^2$, $X^3$ oder $X^4$ nicht die Bedeutung H aufweist.

-C≡C-C≡C-$R^3$ bedeutet bevorzugt -C≡-C-C≡C-C(Alkyl*)$_3$, -C≡C-C≡C-C(Alkyl*)(Alkyl**)$_2$ oder -C≡C-C≡C-CH(Alkyl*)$_2$, insbesondere -C≡C-C≡C-C$(CH_3)_3$, -C≡C-C≡C-CH$(CH_3)_2$ oder -C≡C-C≡C-C$(CH_3)_2C_3H_7$. Alkyl* und Alkyl** bedeuten jeweils unabhängig voneinander $CH_3$, $C_2H_5$ oder $C_3H_7$.

$A^1$ und/oder $A^2$ bedeuten bevorzugt Cyc oder Dio.

Bevorzugt sind auch Verbindungen der Formel I sowie aller Teilformeln, in denen $A^1$ und/oder $A^2$ ein- oder zweifach durch F oder CN substituiertes Cyclohexan-1,4-diyl bedeutet.

Vorzugsweise bedeutet $A^1$ und/oder $A^2$

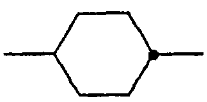 , 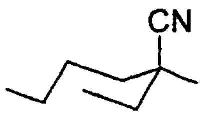 , 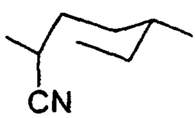 , 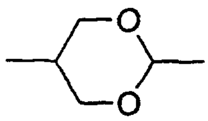 ,

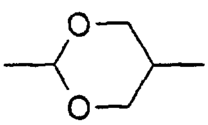 , 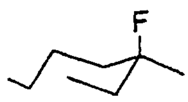 oder 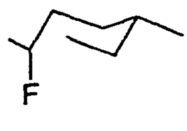 .

m und n sind vorzugsweise 0, 1 oder 2, insbesondere 0 oder 1. m + n ist bevorzugt 1 oder 2.

$Z^1$ und $Z^2$ bedeuten unabhängig voneinander bevorzugt -$CH_2CH_2$-, -$CF_2O$-, -$OCF_2$-, -COO-, -OOC- oder eine Einfachbindung, insbesondere bevorzugt eine Einfachbindung oder -$CH_2$-$CH_2$-.

Verbindungen der Formel I sind bevorzugt, in denen $R^1$ und $R^2$ gleichzeitig Alkyl oder Alkoxy mit 1 bis 10 C-Atomen bedeuten.

Weiterhin sind Verbindungen der Fomel I bevorzugt, worin $Y^1$ und $Y^2$ das Kohlenstoffatom bedeuten. Verbindungen der Formel I, die nicht mehr als einen Dioxanring enthalten, stellen ebenfalls eine bevorzugte Ausführungsform

der Erfindung dar.

Insbesondere sind ferner die Verbindungen der Formeln I1 bis I21 der folgenden Gruppe bevorzugt:

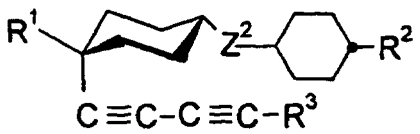

I1

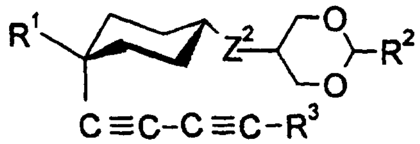

I2

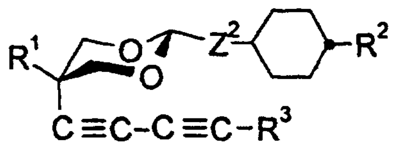

I3

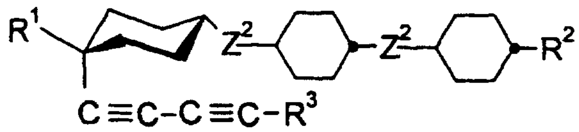

I4

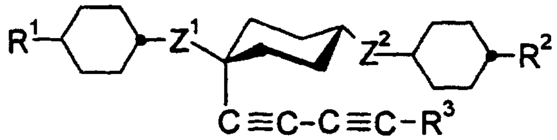

I5

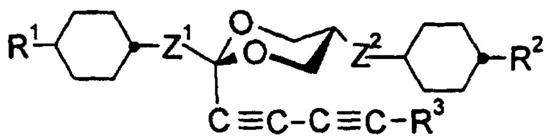

I6

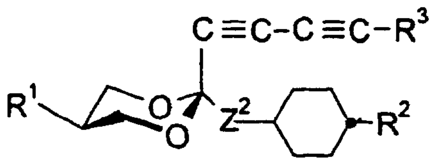

I7

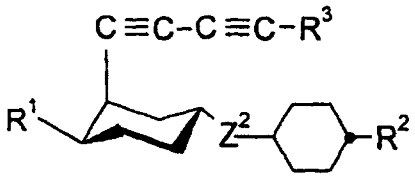

I8

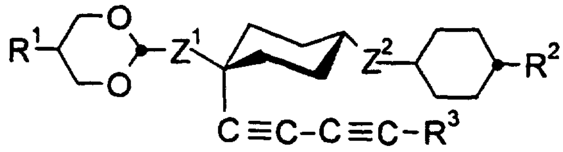

I9

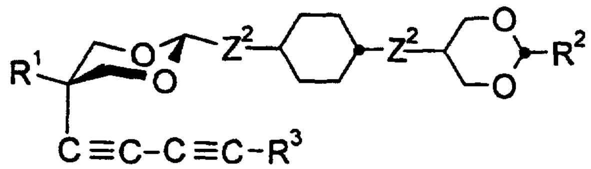

I10

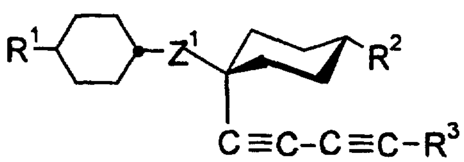

I11

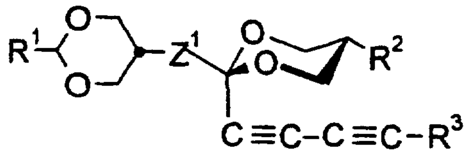

I12

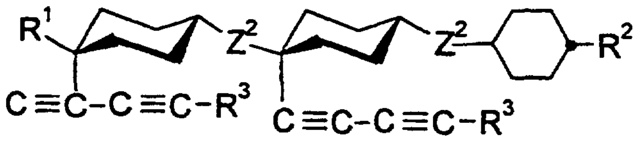

I13

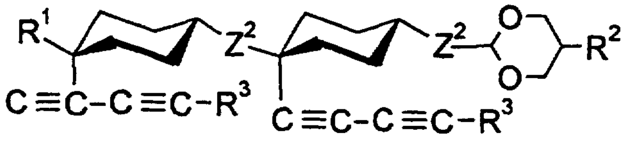

I14

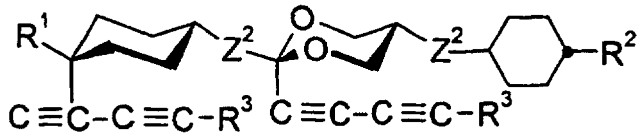

I15

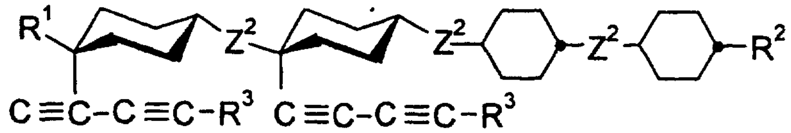

I16

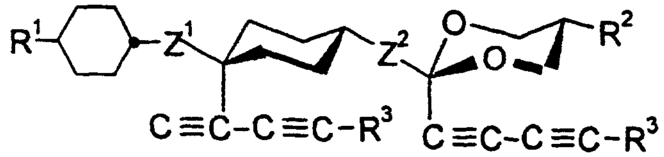

I17

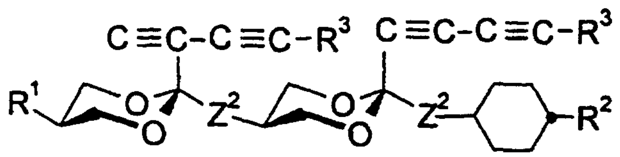

I18

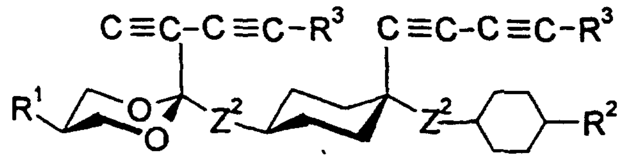

I19

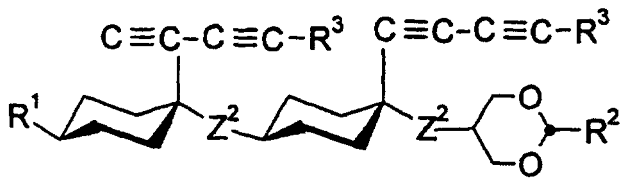

I20

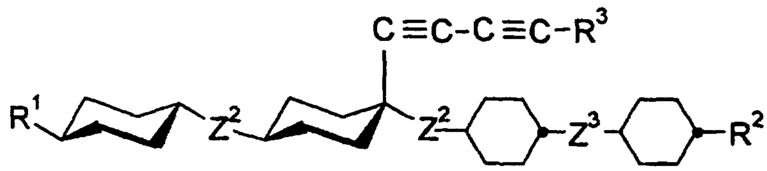

I21

worin $R^1$, $R^2$, $R^3$, $Z^1$, und $Z^2$ die oben angegebenen Bedeutungen aufweisen.

Falls $R^1$ und/oder $R^2$ in den vor- und nachstehenden Formeln einen Alkylrest bedeutet, so kann dieser geradkettig oder verzweigt sein. Vorzugsweise ist er geradkettig, hat 2, 3, 4, 5, 6 oder 7 C-Atome und bedeutet demnach

bevorzugt Ethyl, Propyl, Butyl, Pentyl, Hexyl oder Heptyl, ferner Methyl, Octyl, Nonyl, Decyl, Undecyl, Dodecyl, Tridecyl, Tetradecyl oder Pentadecyl.

Falls $R^1$ und/oder $R^2$ einen Alkylrest bedeutet, in dem eine $CH_2$-Gruppe durch -O- ersetzt ist, so kann dieser geradkettig oder verzweigt sein. Vorzugsweise ist er geradkettig und hat 1 bis 10 C-Atome. Bevorzugt ist die erste $CH_2$-Gruppe dieses Alkylrestes durch -O- ersetzt, so dass der Rest $R^1$ die Bedeutung Alkoxy erhält und vorzugsweise Methoxy, Ethoxy, Propoxy, Butoxy, Pentyloxy, Hexyloxy, Heptyloxy, Octyloxy, Nonyloxy bedeutet.

Weiterhin kann auch eine $CH_2$-Gruppe an anderer Stelle durch -O- ersetzt sein, so dass der Rest $R^1$ und/oder $R^2$ vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-,4-, 5-, 6-, 7- oder 8-Oxanonyl, 2-, 3-, 4-, 5-, 6-, 7-, 8- oder 9-Oxadecyl bedeutet.

Falls $R^1$ und/oder $R^2$ einen Alkenylrest bedeutet, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Besonders bevorzugt bedeutet $R^1$ und/oder $R^2$ einen Alkenylrest aus der folgenden Gruppe:

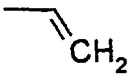, 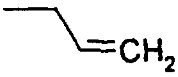, 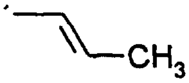, 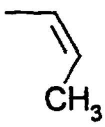, 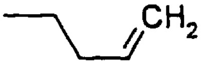,

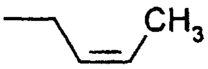, 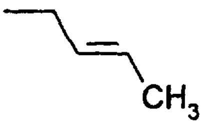, 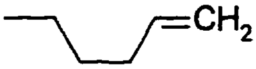, 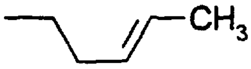,

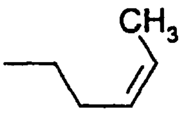, 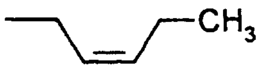, 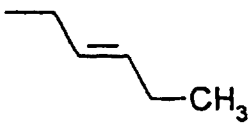,

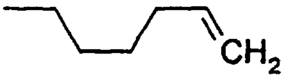, 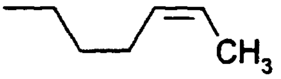, 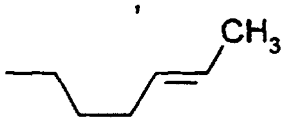,

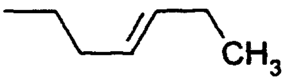, 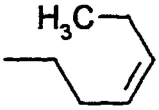, 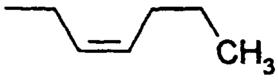,

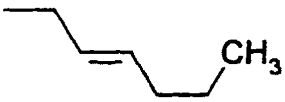.

Falls $R^1$ und/oder $R^2$ einen Alkenyloxyrest bedeutet, so kann dieser geradkettig oder verzweigt sein. Vorzugsweise ist er geradkettig und hat 2 bis 10 C-Atome. Er bedeutet besonders bevorzugt einen Rest der folgenden Gruppe:

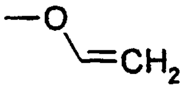, 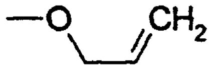, 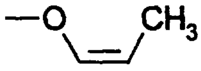, 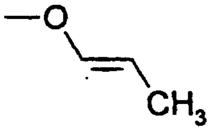,

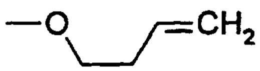, 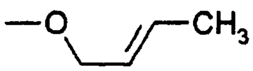, 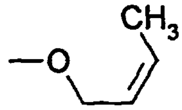, 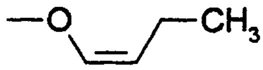,

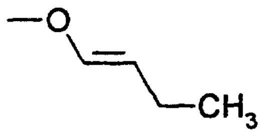, 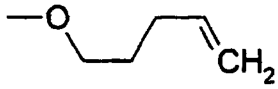, 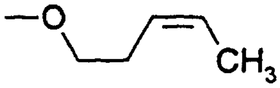,

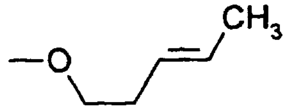, 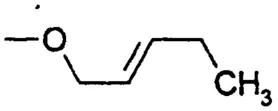, 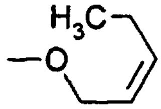,

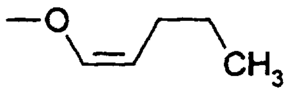; 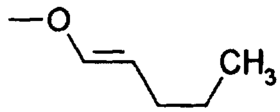.

Falls $R^1$ und/oder $R^2$ einen Alkylrest bedeutet, in dem eine $CH_2$-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)butyl.

Falls $R^1$, und/oder $R^2$ einen mindestens einfach durch Halogen substituierten Alkylrest bedeutet, so ist dieser Rest vorzugsweise geradkettig. Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I mit verzweigter Flügelgruppe $R^1$ und/oder $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste $R^1$ und/oder $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentyloxy, 3-Methylpentyloxy, 2-Ethylhexyloxy, 1-Methylhexyloxy, 1-Methylheptyloxy.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

Einige ganz besonders bevorzugte kleinere Gruppen von Verbindungen der Formel I sind diejenigen der Teilformeln I22 bis I36:

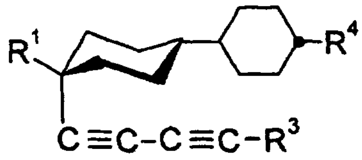

I22

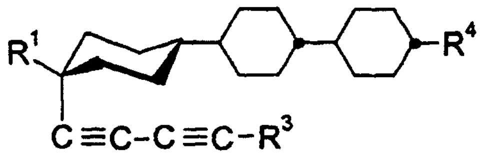

I23

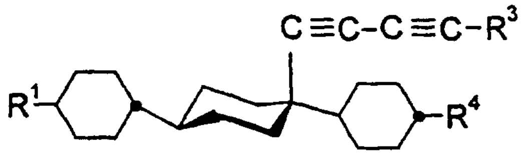

I24

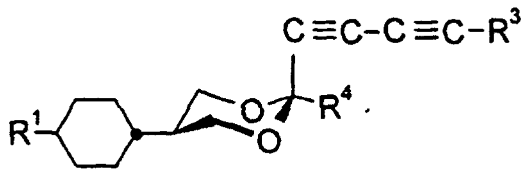

I25

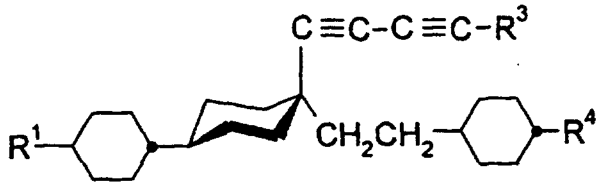

I26

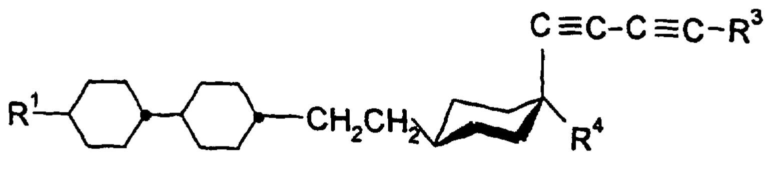

I27

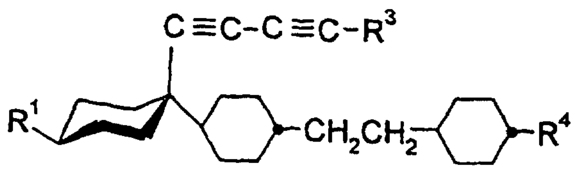

I28

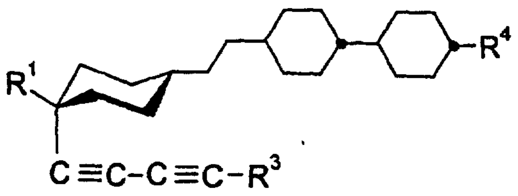

I29

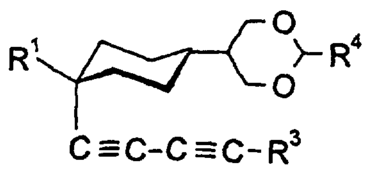

I30

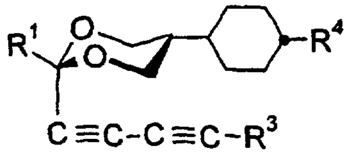

I31

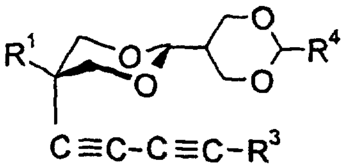

I32

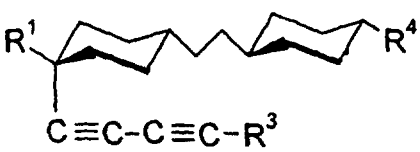

I33

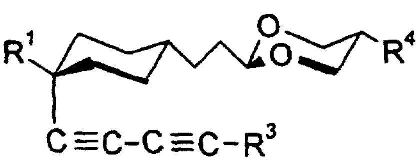

I34

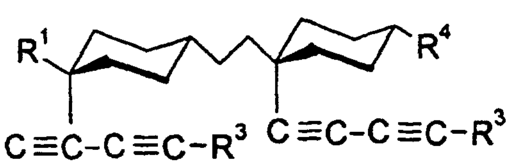

I35

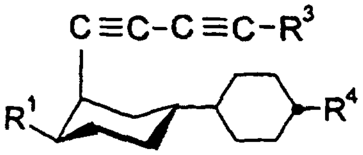

I36

worin $R^1$ die oben angegebene Bedeutung aufweist und $R^4$ Alkyl, Alkenyl oder Alkoxy bedeutet.

Ganz besonders bevorzugte Verbindungen dieser Gruppe sind die der Formeln I22, I23, I25, I26, I28 und I29.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind.

Dabei kann man von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

Die Synthese der Verbindungen der Formel I worin $A^1$ und/oder $A^2$ axial fluoriertes Cyclohexan bedeutet, kann durch Anwendung von Fluorwasserstoff unter Druck oder durch Amin-Fluorwasserstoff-Addukte bewirkt werden (z.B. A. V. Grosse, C. B. Linn, J. Org. Chem. 3, (1938) 26; G. A. Olah, M. Nojima, I. Kerekes, Synthesis, (1973) 779); G. A. Olah, X-Y. Li, Q. Wang, G. K. S. Prakash, Synthesis (1993) 693).

Die erfindungsgemäßen Verbindungen können z.B. nach folgenden Reaktionsschemata hergestellt werden:

Schema 1

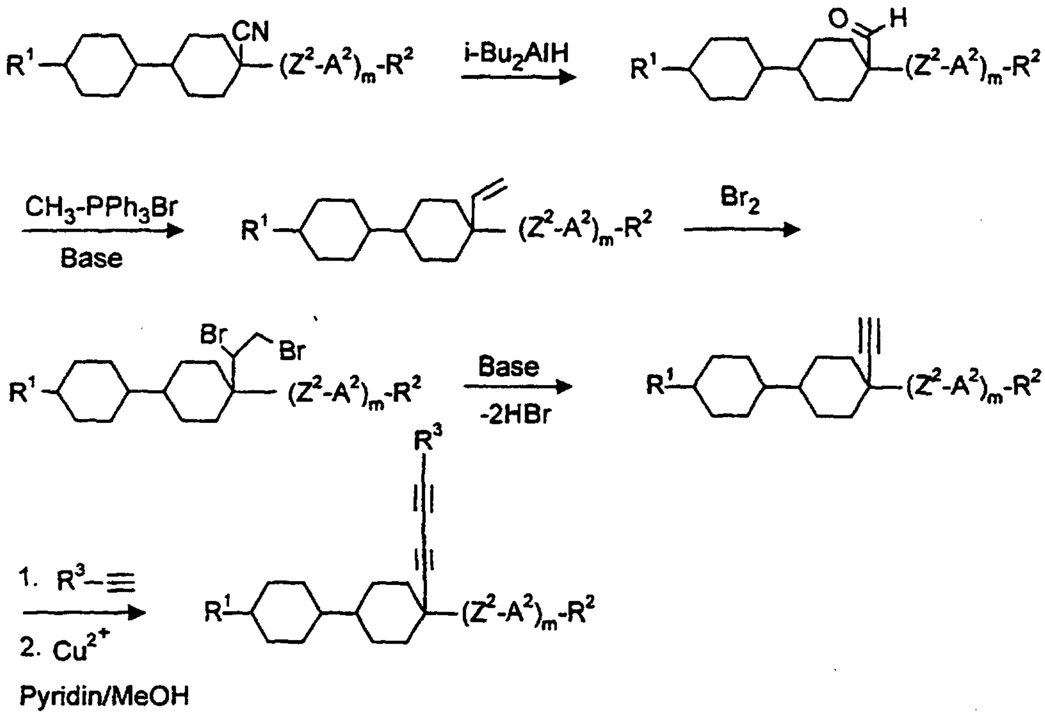

Schema 2

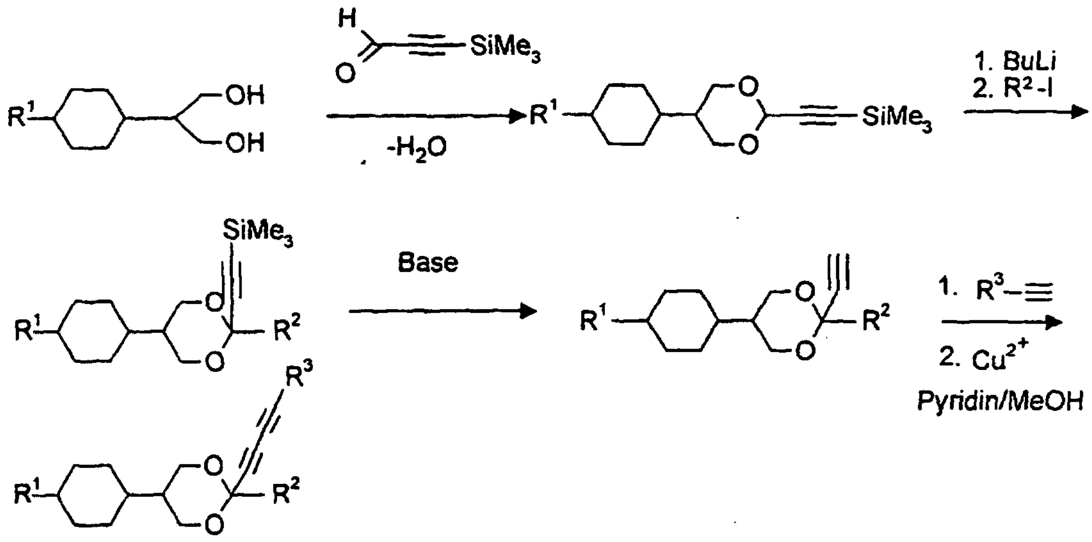


Schema 3

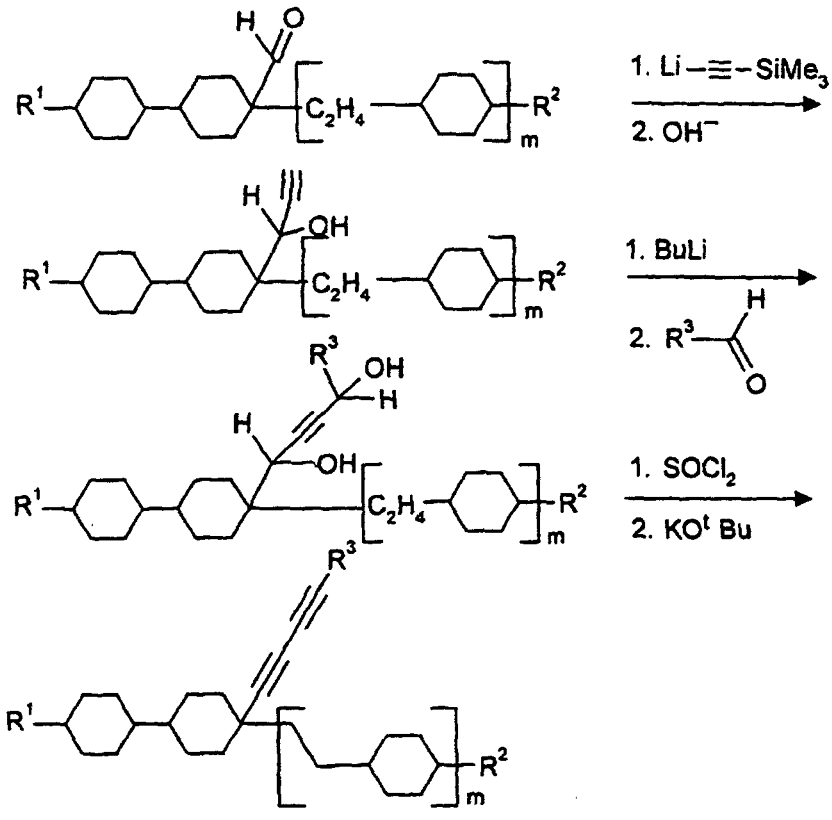


Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC =

Dicyclohexylcarbodiimid) erhalten werden.

Die entsprechenden Carbonsäuren und Alkohole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1-4 C-Atomen in der Alkylgruppe.

Als reaktionsfähige Derivate der genannten Alkohole kommen insbesondere die entsprechenden Metallalkoholate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin, als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50 °C und +250 °C, vorzugsweise zwischen -20 °C und +80 °C. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium-oder Kaliumacetat, Erdalkalimetall- hydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, dass man den Alkohol zunächst in das Natrium- oder Kaliumalkoholat überführt, z.B. durch Behandlung mit ethanolischer Natron- oder Kalilauge, dieses isoliert und mit einem Säureanhydrid oder insbesondere Säurechlorid umsetzt.

Nitrile können durch Austausch von Halogenen mit Kupfercyanid oder Alkalicyanid erhalten werden.

Ether der Formel I sind durch Veretherung entsprechender Hydroxyver bindungen, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat übergeführt wird. Dieses kann dann mit dem entsprechenden Alkylhalogenid, -sulfonat oder Dialkylsulfat umgesetzt werden, zweckmäßig in einem inerten Lösungsmittel, wie z.B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20 °C und 100 °C.

Die metallorganischen Verbindungen stellt man beispielsweise durch Metall-Halogenaustausch (z.B. nach Org. React. 6, 339-366 (1951)) zwischen der entsprechenden Halogen-Verbindung und einer lithiumrganischen Verbindung wie vorzugsweise tert-Butyllithium oder Lithium-Naphthalenid oder durch Umsatz mit Magnesiumspänen her.

Darüberhinaus können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion sind Verbindungen entsprechend der Formel I, die aber an Stelle eines Cyclohexanringes einen Cyclohexenring oder Cyclohexanonring und/oder an Stelle einer $-CH_2CH_2$-Gruppe eine -CH=CH-Gruppe und/oder an Stelle einer $-CH_2$-Gruppe eine -CO-Gruppe und/oder an Stelle eine H-Atoms eine freie oder eine funktionell (z.B. in Form ihres p-Toluolsuffonats) abgewandelte OH-Gruppe enthalten.

Die Reduktion kann z.B. erfolgen durch katalytische Hydrierung bei Temperaturen zwischen etwa 0 °C und etwa 200 °C sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen

zwischen etwa 80 und 120 °C) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperturen zwischen etwa 100 und 200 °C) zu den entsprechendenn Verbindungen der Formel I, die Alkylgruppen und/oder $-CH_2CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100 °C.

Doppelbindungen können mit $NaBH_4$ oder Tributylzinnhydrid in Methanol hydriert werden.

Die Ausgangsmaterialien sind entweder bekannt oder können in Analogie zu bekannten Verbindungen hergestellt werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1 ,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane, Naphthaline, Dekaline und substituierten Zimtsäuren. Die 1,4-Phenylengruppen in diesen Verbindungen können auch fluoriert sein.

Die wichtigsten als weitere Bestandteile efindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-$CH_2CH_2$-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-,- Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2-5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy, Alkenyloxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder $-(O)_i\ CH_{3-(k+l)}\ F_kCl_l$, wobei i 0 oder 1 und k und l1, 2 oder 3 sind; die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1 b, 2b, 3b, 4b und 5b, in denen R" die Bedeutung -F, -Cl, -NCS, $-CF_3$, $-OCHF_2$ oder $-OCF_3$ hat.

In den Verbindungen der Teilformeln 1b, 2b, 3b, 4b und 5b hat R' die bei den Verbindungen der Teilformeln 1 a-5a angegebene Bedeutung und ist vorzugsweise Alkyl, Alkenyl, Alkoxy, Alkenyloxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformein 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise:

| | |
|---|---|
| Gruppe A: | 0 bis 90 %, vorzugsweise 20 bis 90 %, insbesondere 30 bis 90 % |
| Gruppe B: | 0 bis 80 %, vorzugsweise 10 bis 80 %, insbesondere 10 bis 65 % |
| Gruppe C: | 0 bis 80 %, vorzugsweise 5 bis 80 %, insbesondere 5 bis 50 % |

wobei die Summe der Massenanteile der in den jeweiligen erfindungsgemäßen Medien enthaltenen Verbindungen aus den Gruppen A und/oder B und/oder C vorzugsweise 5 bis 90 % und insbesondere 10 bis 90 % beträgt.

Die erfindungsgemäßen Medien enthalten vorzugsweise 1 bis 40 %, insbesondere vorzugsweise 5 bis 30 %, der erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise ein oder zwei, ferner drei bis vier erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder Orientierung der nematischen Phasen zugesetzt werden.

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt Klp. = Klärpunkt. Ferner bedeuten K = kristalliner Zustand, N = nematische Phase, Sm = smektische Phase und I = isotrope Phase. Die Angaben zwischen diesen Symbolen stellen die Übergangstemperaturen dar. $\Delta n$ bedeutet optische Anisotropie (589 nm 20 °C) und $\Delta\varepsilon$ die dielektrische Anisotropie (1kHz, 20 °C).

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie.

Folgende Abkürzungen werden verwendet:

| | |
|---|---|
| THF | Tetrahydrofuran |
| KOtBu | Kalium-tert.-butylat |
| MTB-Ether | Methyl-tert.-butylether |
| DMSO | Dimethylsulfoxid |

Beispiele

Beispiel 1

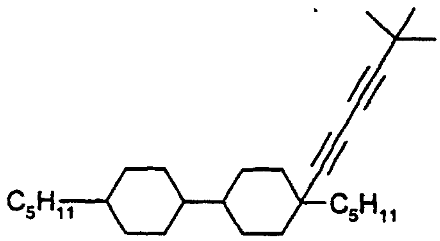

Schritt 1.1

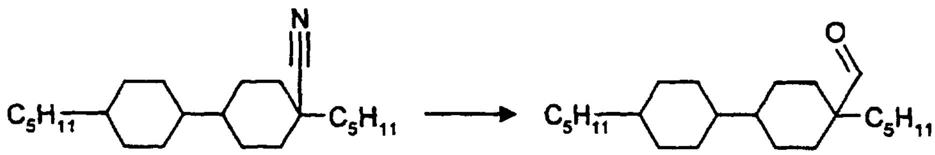

A B

640 mmol A werden in 800 ml Toluol gelöst und zu diesem Gemisch werden 755 mmol Diisobutylaluminiumhydrid zugetropft. Es wird ca. 2 h bei Raumtemperatur gerührt. Das Reaktionsgemisch auf Eis/HCl gegossen, das entstehende 2-Phasen Gemisch ca. 1 h gut gerührt, damit das entstehende Imin zum Aldehyd hydrolisiert wird. Das Reaktionsgemisch wird mit Toluol extrahiert, die vereinigten organischen Phasen mit Wasser gewaschen, über Natriumsulfat getrocknet, filtriert und wie üblich aufgearbeitet.

Schritt 1.2

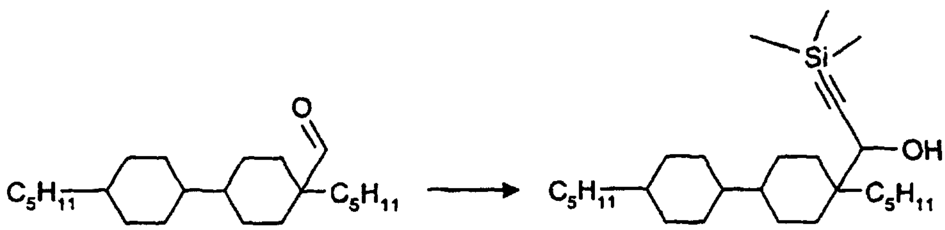

B C

651 mmol (Trimethylsilyl)acetylen werden in 300 ml THF abs. gelöst und bei 0 bis -10 °C werden 651 mmol BuLi (15 %ige Lösung in n-Hexan) zugetropft. Nach ca. 10 min Rühren werden 542 mmol B gelöst in 350 ml abs. THF bei 0 bis -10 °C zugetropft. Man rührt 1 h, versetzt vorsichtig mit Wasser und arbeitet wie üblich auf.

Schritt 1.3

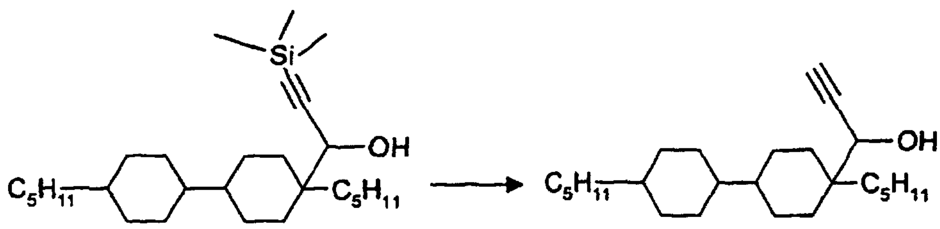

C D

0,54 mol C werden mit 1,3 mol KOH in 1500 ml Methanol gelöst und versetzt. Das Reaktionsgemisch wird über Nacht bei Raumtemperatur gerührt, mit Wasser versetzt und mit HCl neutralisiert. Zuletzt wird wie üblich aufgearbeitet.

Schritt 1.4

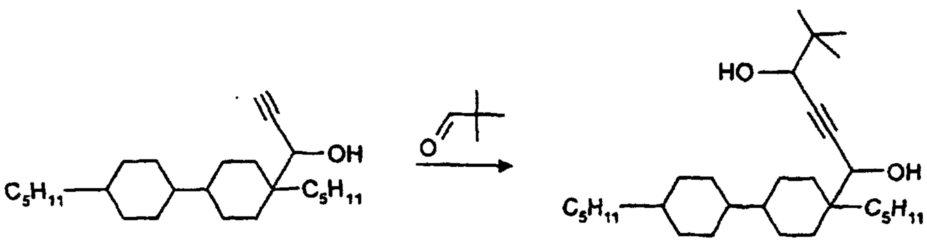

D E

89 mmol D in 100 ml abs. THF vorgelegt werden und bei 0 bis -10 °C wird mit 179 mmol BuLi (15 %ige Lösung in n-Hexan) versetzt. Man rührt 10 min und versetzt das Reaktionsgemisch bei 0 bis -10 °C mit 89 mmol Pivalinaldehyd gelöst in 40 ml abs. THF. Das Reaktionsgemisch wird 1h gerührt mit Wasser versetzt, mit verd. HCl angesäuert und wie üblich aufgearbeitet.

Schritt 1.5

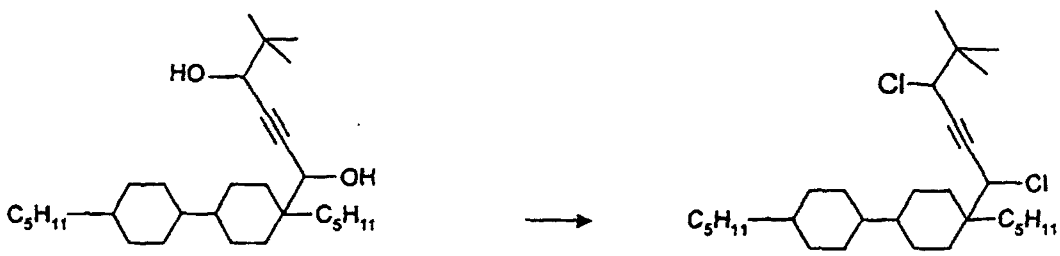

E F

60 mmol E gelöst in 100 ml Dichlormethanol werden bei Raumtemperatur mit 180 mmol Thionylchlorid versetzt. Nach 48h Rühren wird das Reaktionsgemisch mit Wasser versetzt und wie üblich aufgearbeitet.

Schritt 1.6

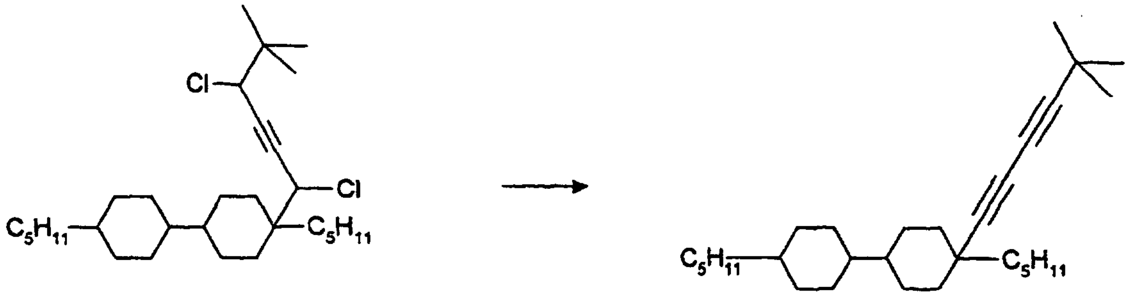

F G

50 mmol F werden in 100 ml abs. DMSO gelöst, portionsweise mit 150 mmol KO$^t$Bu versetzt und 2h bei 120 °C gerührt. Man läßt das Reaktionsgemisch abkühlen und versetzt vorsichtig mit verd. HCl. Zuletzt wird wie üblich aufgearbeitet. Das Produkt wird aus n-Hexan/i-Propanol (1:10) umkristallisiert.
K 84 N (23,6)I; $\Delta n$ = -0,059; $\Delta\varepsilon$ = -3,8

Analog werden unter Verwendung der entsprechenden Vorstufen die folgenden erfindungsgemäßen Verbindungen hergestellt:

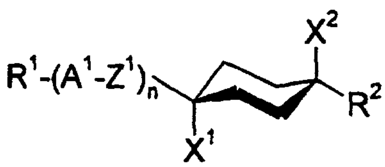

| $R^1$ | $(A^1-Z^1)_n$ | $X^2$ | $X^1$ | $R^2$ |
|---|---|---|---|---|
| n-Pentyloxy | 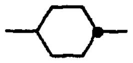 | $-C\equiv C-C\equiv C-CH(CH_3)_2$ | H | n-Propyl |
| n-Propyl | 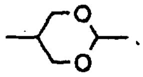 | $-C\equiv C-C\equiv C-CH_3$ | H | $OCF_3$ |
| n-Pentyl | 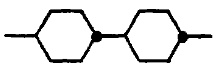 | $-C\equiv C-C\equiv C-CH_3$ | H | $CF_3$ |
| Ethoxy | 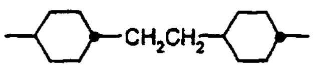 | $-C\equiv C-C\equiv C-CF_3$ | H | F |
| n-Pentyl | 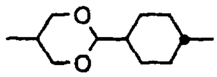 | $-C\equiv C-C\equiv C-C(CH_3)_3$ | H | n-Pentyloxy |
| n-Pentyl | 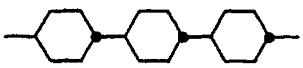 | $-C\equiv C-C\equiv C-CH_3$ | H | $CF_3CF_3$ |
| H | 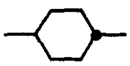 | $-C\equiv C-C\equiv C-C(CH_3)_3$ | H | n-Propyl |
| Pentyloxy | 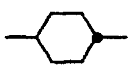 | $-C\equiv C-C\equiv C-CF_3$ | H | $CHFCF_3$ |
| n-Pentyl | 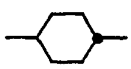 | H | $-C\equiv C-C\equiv C-CF_3$ | n-Propyl |
| n-Propyl | 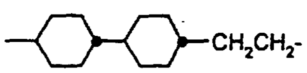 | H | $-C\equiv C-C\equiv C-C(CH_3)_3$ | n-Propyl |
| n-Propyl | 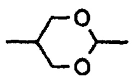 | H | $-C\equiv C-C\equiv C-CF_3$ | n-Propyl |
| n-Propyl | 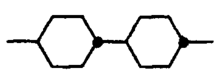 | H | $-C\equiv C-C\equiv C-C(CH_3)_3$ | n-Propyl |
| Ethoxy | 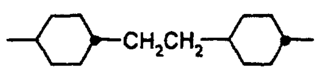 | $-C\equiv C-C\equiv C-CH_3$ | $-C\equiv C-C\equiv C-CH_3$ | Methyl |
| Hexyloxy | 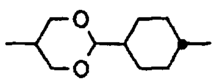 | H | $-C\equiv C-C\equiv C-CF_3$ | n-Propyloxy |
| n-Pentyl | 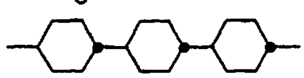 | $-C\equiv CH$ | $-C\equiv C-C\equiv C-C(CH_3)_3$ | n-Propyloxy |
| n-Propyl | 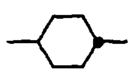 | H | $-C\equiv C-C\equiv C-C(CH_3)_3$ | n-Propyl |
| n-Propyl | 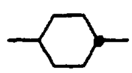 | H | $-C\equiv C-C\equiv C-C(C_3H_7)(CH_3)_2$ | n-Propyl |

| $R^1$ | $(A^1-Z^1)_n$ | $X^2$ | $X^1$ | $R^2$ |
|---|---|---|---|---|
| n-Pentyloxy | 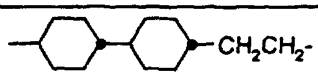 | H | $-C\equiv C-C\equiv C-CF_3$ | Methyl |
| n-Pentyl | 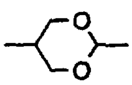 | H | $-C\equiv C-C\equiv C-C(CH_3)_3$ | n-Pentyl |

Analog werden unter Verwendung der entsprechenden Vorstufen die folgenden erfindungsgemäßen Verbindungen hergestellt:

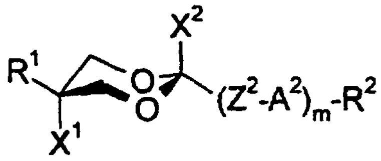

| $R^1$ | $(Z^2-A^2)_n$ | $X^1$ | $X^2$ | $R^2$ |
|---|---|---|---|---|
| n-Propyl | 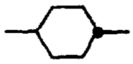 | $-C{\equiv}C-C{\equiv}C-C(CH_3)_3$ | H | $CHFCF_3$ |
| n-Pentyloxy | 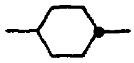 | $-C{\equiv}C-C{\equiv}C-Cl$ | H | n-Propyl |
| n-Propyl | 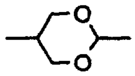 | $-C{\equiv}C-C{\equiv}C-CF_3$ | H | $OCF_3$ |
| n-Pentyl | 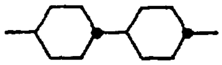 | $-C{\equiv}C-C{\equiv}C-C(CH_3)_3$ | H | $CF_3$ |
| Ethoxy | 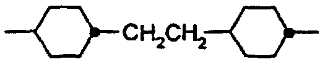 | $-C{\equiv}C-C{\equiv}C-C(CH_3)_3$ | H | F |
| n-Hexyloxy | 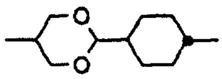 | $-C{\equiv}C-C{\equiv}C-Cl$ | H | n-Propyloxy |
| n-Pentyl | 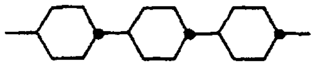 | $-C{\equiv}C-C{\equiv}C-CH_3$ | H | $CF_2CF_3$ |
| n-Propyl | 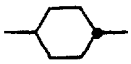 | $-C{\equiv}C-C{\equiv}C-C(CH_3)_3$ | H | n-Propyl |
| n-Pentyloxy | 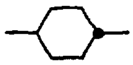 | $-C{\equiv}C-C{\equiv}C-C(CH_3)_3$ | H | $CHFCF_3$ |
| n-Propyl | 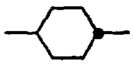 | $-C{\equiv}C-C{\equiv}C-Cl$ | H | $OCF_3$ |

Analog werden unter Verwendung der entsprechenden Vorstufen die folgenden erfindungsgemäßen Verbindungen hergestellt:

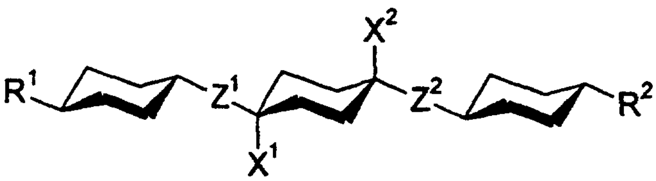

| $R^1$ | $Z^1$ | $Z^2$ | $X^1$ | $X^2$ | $R^2$ |
|---|---|---|---|---|---|
| n-Pentyloxy | ——— | ——— | $-C{\equiv}C-C{\equiv}C-C(CH_3)_3$ | H | $CHFCF_3$ |
| n-Propyl | ——— | ——— | $-C{\equiv}C-C{\equiv}C-C(CH_3)_3$ | H | n-Propyl |
| n-Propyl | $-CH_2CH_2-$ | ——— | $-C{\equiv}C-C{\equiv}CF_3$ | H | n-Propyl |
| n-Pentyl | ——— | -COO- | $-C{\equiv}C-C{\equiv}C-CH_3$ | H | $CF_3$ |
| n-Propyl | $-CH_2CH_2-$ | $-CH_2CH_2-$ | $-C{\equiv}C-C{\equiv}C-C(CH_3)_3$ | $-C{\equiv}C-C{\equiv}C-Cl$ | n-Propyl |
| n-Hexyloxy | $-CH_2CH_2-$ | ——— | $-C{\equiv}C-C{\equiv}C-C(CH_3)_3$ | H | n-Propyloxy |

Tabelle fortgesetzt

| $R^1$ | $Z^1$ | $Z^2$ | $X^1$ | $X^2$ | $R^2$ |
|---|---|---|---|---|---|
| n-Pentyl | ——— | ——— | -C≡C-C≡C-Cl | H | $CF_2CF_3$ |
| n-Propyl | ——— | ——— | -C≡C-C≡C-$C(CH_3)_3$ | -C≡C-C≡$SF_5$ | n-Propyl |
| n-Pentyloxy | ——— | ——— | -C≡C-C≡C-$C(CH_3)_3$ | H | $CHFCF_3$ |
| n-Propyl | -OOC- | ——— | -C≡C-C≡C-$CH_3$ | H | $OCF_3$ |
| n-Propyl | -OOC- | -$CH_2CH_2$- | H | -C≡C-C≡C-$CF_3$ | $CHFCF_3$ |
| n-Pentyloxy | ——— | ——— | H | -C≡C-C≡C-$C(CH_3)_3$ | n-Pentyl |
| n-Propyl | ——— | -OOC- | H | -C≡C-C≡C-$CH_3$ | n-Propyl |
| n-Pentyl | -$CH_2CH_2$- | ——— | H | -C≡C-C≡C-$C(CH_3)_3$ | n-Propyl |
| Ethoxy | ——— | ——— | H | -C≡C-C≡C-$CH_3$ | Methyl |
| n-Hexyloxy | ——— | -$CH_2CH_2$- | H | -C≡C-C≡C-$C(CH_3)_3$ | n-Propyloxy |
| n-Pentyl | ——— | ——— | H | -C≡C-C≡C-Cl | n-Propyloxy |
| n-Propyl | $CF_2O$- | ——— | H | -C≡C-C≡C-H | n-Propyl |
| n-Propyl | ——— | -$CF_2O$- | H | -C≡C-C≡C-H | n-Propyl |
| n-Pentyl | -$CF_2O$- | ——— | H | -C≡C-C≡C-H | n-Pentyl |
| n-Pentyl | ——— | -$CF_2O$- | H | -C≡C-C≡C-H | n-Pentyl |
| n-Propyl | ——— | -$CH_2CH_2$- | H | -C≡C-C≡C-$C(CH_3)_3$ | n-Propyl |
| n-Pentyloxy | -OOC- | ——— | H | -C≡C-C≡C-$C(CH_3)_3$ | Methyl |
| n-Pentyl | ——— | ——— | H | -C≡C-C≡C-$CH_3$ | n-Pentyl |

Analog werden unter Verwendung der entsprechenden Vorstufen die folgenden erfindungsgemäßen Verbindungen hergestellt:

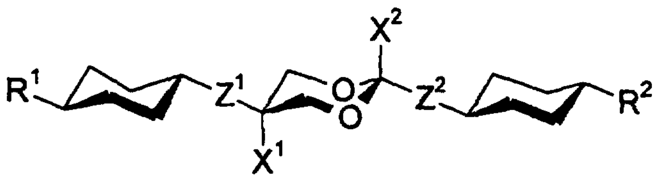

| $R^1$ | $Z^1$ | $Z^2$ | $X^1$ | $X^2$ | $R^2$ |
|---|---|---|---|---|---|
| n-Pentyloxy | ——— | ——— | -C≡C-C≡C-$C(CH_3)_3$ | H | $CHFCF_3$ |
| n-Pentyloxy | ——— | ——— | -C≡C-C≡C-Cl | | n-Propyl |
| n-Propyl | ——— | -$CH_2CH_2$- | -C≡C-C≡C-Cl | -C≡C-C≡C-Cl | $OCF_3$ |
| n-Pentyl | ——— | -COO- | -C≡C-C≡C-CH | H | $CF_3$ |
| Ethoxy | -$CH_2CH_2$- | -$CH_2CH_2$- | -C≡C-C≡C-$C(CH_3)_3$ | H | F |
| n-Hexyloxy | -$CH_2CH_2$- | ——— | -C≡C-C≡C-Cl | H | n-Propyloxy |
| n-Pentyl | ——— | ——— | -C≡C-C≡C-$CF_3$ | -C≡C-C≡C-$CF_3$ | $CF_2CF_3$ |
| n-Propyl | ——— | ——— | -C≡C-C≡C-$C(CH_3)_3$ | H | n-Propyl |
| n-Pentyloxy | ——— | ——— | -C≡C-C≡C-$C(CH_3)_3$ | H | $CHFCF_3$ |
| n-Propyl | ——— | -OOC- | -C≡C-C≡C-$C(CH_3)_3$ | H | $OCF_3$ |
| n-Propyl | -OOC- | -$CH_2CH_2$- | H | -C≡C-C≡C-$CH_3$ | $CHFCF_3$ |
| n-Pentytoxy | ——— | ——— | -C≡C-C≡C-$C(CH_3)_3$ | -C≡C-C≡C-$C(CH_3)_3$ | n-Pentyl |
| n-Propyl | ——— | ——— | H | -C≡C-C≡C-$C(CH_3)_3$ | n-Propyl |
| n-Pentyl | -$CH_2CH_2$- | ——— | H | -C≡C-C≡C-Cl | n-Propyl |
| n-Propyl | -$CF_2O$- | ——— | H | -C≡C-C≡C-H | n-Propyl |

Tabelle fortgesetzt

| $R^1$ | $Z^1$ | $Z^2$ | $X^1$ | $X^2$ | $R^2$ |
|---|---|---|---|---|---|
| n-Propyl | ——— | $-CF_2O-$ | H | -C≡C-C≡C-H | n-Propyl |
| n-Propyl | $-CF_2O-$ | ——— | H | -C≡C-C≡C-H | n-Pentyl |
| n-Propyl | ——— | $-CF_2O-$ | H | -C≡C-C≡C-H | n-Pentyl |
| Ethoxy | ——— | ——— | $-C≡C-C≡C-C(CH_3)_3$ | $-C≡C-C≡C-C(CH_3)_3$ | Methyl |
| n-Hexyloxy | ——— | $-CH_2CH_2-$ | H | -C≡C-C≡C-Cl | n-Propytoxy |
| n-Pentyl | ——— | ——— | H | $-C≡C-C≡C-C(CH_3)_3$ | n-Propyloxy |
| n-Propyl | $-CH_2CH_2-$ | ——— | H | $-C≡C-C≡C-C(CH_3)_3$ | n-Propyl |
| n-Pentyloxy | -OOC- | ——— | H | $-C≡C-C≡C-C(CH_3)_3$ | Methyl |
| n-Pentyl | ——— | ——— | H | -C≡C-C≡C-Cl | n-Pentyl |

Analog werden unter Verwendung der entsprechenden Vorstufen die folgenden erfindungegemäßen Verbindungen erhalten:

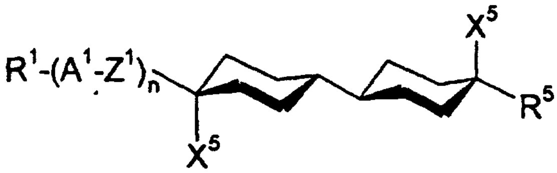

| $R^1$ | $(A^1-Z^1)_n$ | $X^5$ | $R^5$ |
|---|---|---|---|
| n-Propyl | 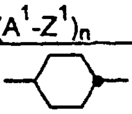COO- | $-C≡C-C≡C-C(CH_3)_3$ | -OOC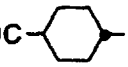n-Propyl |
| n-Pentyloxy | 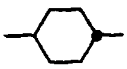COO- | $-C≡C-C≡C-CH_3$ | n-Propyloxy |
| n-Propyloxy | ——— | $-C≡C-C≡C-CF_3$ | $OCF_3$ |

| $R^1$ | $(A^1-Z^1)_n$ | $X^5$ | $R^5$ |
|---|---|---|---|
| n-Pentyl | 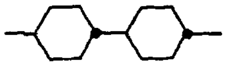 COO- | -C≡C-C≡C-C(CH₃)₃ | $OCH_3$ |
| Ethyl | 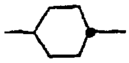 $CH_2O$- | -C≡C-C≡C-C(CH₃)₃ | n-Propyloxy |
| n-Hexyl | 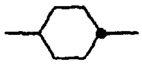 COO- | -C≡C-C≡C-C(CH₃)₃ | -OOC 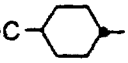 Methyl |
| n-Pentyloxy | — | -C≡C-C≡C-Cl | $OCF_2CF_3$ |
| n-Propyl | 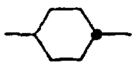 COO- | -C≡C-C≡C-C(CH₃)₃ | n-Pentyloxy |
| n-Butyl | 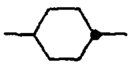 COO- | -C≡C-C≡C-C(CH₃)₃ | $OCF=CF_2$ |
| n-Propyloxy | — | -C≡C-C≡C-C(CH₃)₃ | $OCF_3$ |
| n-Propyl | 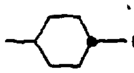 $CF_2O$- | -C≡C-C≡C-C(CH₃)₃ | n-Propyl |
| n-Propyl | 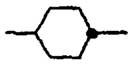 $CF_2O$- | -C≡C-C≡C-C(CH₃)₃ | F |
| n-Propyl | 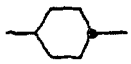 $CF_2O$- | -C≡C-C≡C-C(CH₃)₃ | $OCF_3$ |
| n-Propyl | 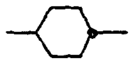 $CF_2O$- | $-C≡C-C≡C-C(C_2H_5)_2C_3H_7$ | $CF_3$ |

In analoger Weise zu den vorhergehenden Beispielen werden die folgenden erfindungsgemäßen Verbindungen hergestellt:

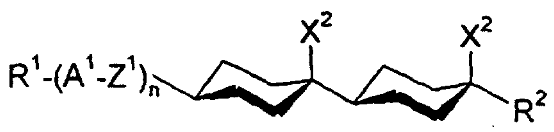

| $R^1$ | $(A^1-Z^1)_n$ | $X^2$ | $R^2$ |
|---|---|---|---|
| n-Propyl | 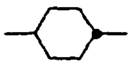 | -C≡C-C≡C-C(CH₃)₃ | $CHFCF_3$ |
| n-Pentyloxy | 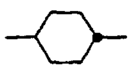 | -C≡C-C≡C-C(CH₃)₃ | n-Propyl |
| n-Propyl | 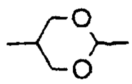 | $-C≡C-C≡C-CH_3$ | $OCF_3$ |
| Ethoxy | 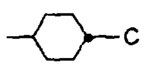 $CH_2CH_2$ 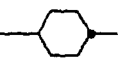 | -C≡C-C≡C-C(CH₃)₃ | F |
| n-Hexyloxy | 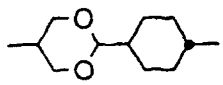 | -C≡C-C≡C-C(CH₃)₃ | n-Propyloxy |

| $R^1$ | $(A^1\text{-}Z^1)_n$ | $X^2$ | $R^2$ |
|---|---|---|---|
| n-Propyl | 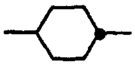 | -C≡C-C≡C-$CF_3$ | n-Propyl |
| n-Pentyloxy | 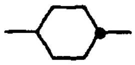 | -C≡C-C≡C-C$(CH_3)_3$ | $CHFCF_3$ |
| n-Propyl | 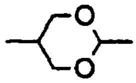 | -C≡C-C≡C-$CH_3$ | $OCF_3$ |
| n-Propyl | 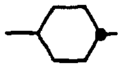 $CF_2O$- | -C≡C-C≡C-$CH_3$ | $CF_3$ |
| n-Propyl | 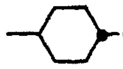 $CF_2O$- | -C≡C-C≡C-$CH_3$ | F |

In analoger Weise zu den vorhergehenden Beispielen werden die folgenden erfindungsgemäßen Verbindungen hergestellt:

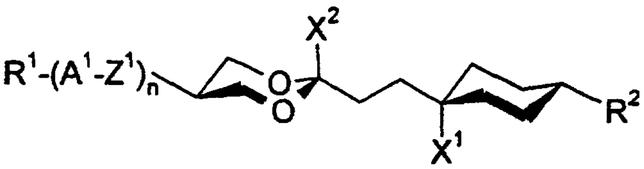

| $R^1$ | $(A^1\text{-}Z^1)_n$ | $X^1$ | $X^2$ | $R^2$ |
|---|---|---|---|---|
| n-Propyl | 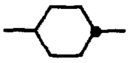 | -C≡C-C≡C-C$(CH_3)_3$ | H | $CHFCF_3$ |
| n-Pentyloxy | 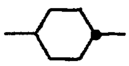 | -C≡C-C≡C-C$(CH_3)_3$ | -C≡C-C≡C-C$(CH_3)_3$ | n-Propyl |
| n-Propyl | 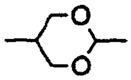 | -C≡C-C≡C-C$(CH_3)_3$ | H | $OCF_3$ |
| Ethoxy | 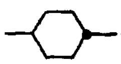 $CH_2CH_2$ 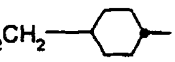 | -C≡C-C≡C-Cl | H | F |
| n-Propyl | 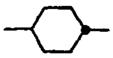 $CF_2O$- | -C≡C-C≡C-H | H | n-Propyl |
| n-Propyl | 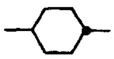 $CF_2O$- | -C≡C-C≡C-H | H | F |
| n-Propyl | 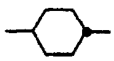 $CF_2O$- | -C≡C-C≡C-H | H | $CF_3$ |
| n-Pentyl | 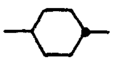 $CF_2O$- | -C≡C-C≡C-H | H | n-Propyl |
| n-Pentyl | 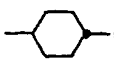 $CF_2O$- | -C≡C-C≡C-H | H | F |
| n-Pentyl | 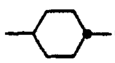 $CF_2O$- | -C≡C-C≡C-H | H | $CF_3$ |
| n-Hexyloxy | 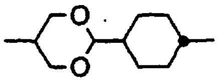 | -C≡C-C≡C-C$(CH_3)_3$ | -C≡C-C≡C-CN | n-Propyloxy |

| $R^1$ | $(A^1-Z^1)_n$ | $X^1$ | $X^2$ | $R^2$ |
|---|---|---|---|---|
| n-Pentyl | 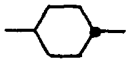 | $-C{\equiv}C-C{\equiv}C-CH_3$ | H | n-Propyl |
| n-Propyloxy | 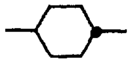 | $-C{\equiv}C-C{\equiv}C-Cl$ | H | $CHFCF_3$ |

In analoger Weise zu den vorhergehenden Beispielen werden die folgenden erfindungsgemäßen Verbindungen hergestellt:

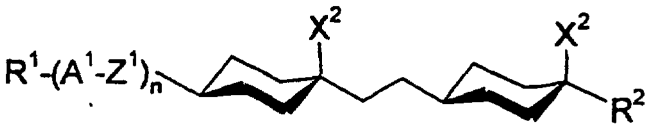

| $R^1$ | $(A^1-Z^1)_n$ | $X^2$ | $R^2$ |
|---|---|---|---|
| n-Propyl | 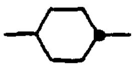 | $-C{\equiv}C-C{\equiv}C-C(CH_3)_3$ | $CHFCF_3$ |
| n-Pentyloxy | 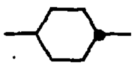 | $-C{\equiv}C-C{\equiv}C-C(CH_3)_3$ | n-Propyl |
| n-Propyl | 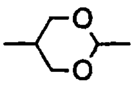 | $-C{\equiv}C-C{\equiv}C-Cl$ | $OCF_3$ |
| n-Hexyloxy | 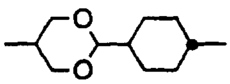 | $-C{\equiv}C-C{\equiv}C-CF_3$ | n-Propyloxy |
| n-Propyl | 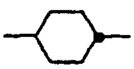 | $-C{\equiv}C-C{\equiv}C-C(CH_3)_3$ | n-Propyl |
| n-Pentyloxy | 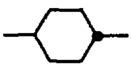 | $-C{\equiv}C-C{\equiv}C-C(CH_3)_3$ | $CHFCF_3$ |
| n-Propyl | 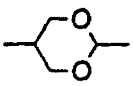 | $-C{\equiv}C-C{\equiv}C-CH_3$ | $OCF_3$ |
| n-Propyl | 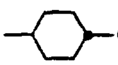 $-CF_2O-$ | $-C{\equiv}C-C{\equiv}C-CH_3$ | n-Propyl |
| n-Propyl | 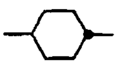 $-CF_2O-$ | $-C{\equiv}C-C{\equiv}C-CH_3$ | F |
| n-Propyl | 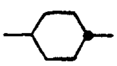 $-CF_2O-$ | $-C{\equiv}C-C{\equiv}C-CH_3$ | $CF_3$ |
| Vinyl | 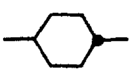 | $-C{\equiv}C-C{\equiv}C-CH_3$ | n-Propyl |
| Vinyl | 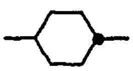 | $-C{\equiv}C-C{\equiv}C-CH(C_2H_5)_2$ | n-Propyl |

In analoger Weise zu den vorhergehenden Beispielen werden die folgenden erfindungsgemäßen Verbindungen hergestellt:

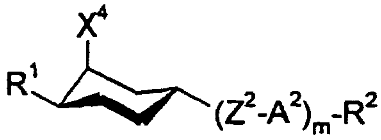

| $R^1$ | $(Z^2-A^2)_m$ | $X^4$ | $R^2$ |
|---|---|---|---|
| n-Pentyloxy | 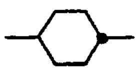 | $-C\equiv C-C\equiv C-C(CH_3)_3$ | n-Propyl |
| n-Propyl | 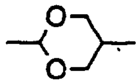 | $-C\equiv C-C\equiv C-CH_3$ | $OCF_3$ |
| n-Pentyl | 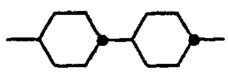 | $-C\equiv C-C\equiv C-C(CH_3)_3$ | $CF_3$ |
| Ethyl | 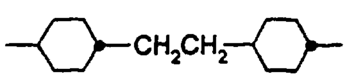 | $-C\equiv C-C\equiv C-CF_3$ | F |
| n-Hexyl | 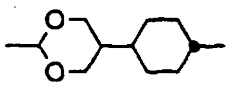 | $-C\equiv C-C\equiv C-C(CH_3)_3$ | n-Propyloxy |
| n-Pentyl | 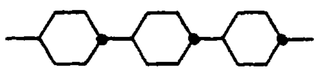 | $-C\equiv C-C\equiv C-CH_3$ | $CF_3CF_3$ |
| n-Pentyl | 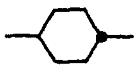 | $-C\equiv C-C\equiv C-CH_3$ | n-Pentyloxy |
| n-Propyloxy | 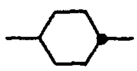 | $-C\equiv C-C\equiv C-CF_3$ | $CHFCF_3$ |

## Patentansprüche

1. Dialkinverbindungen der Formel I

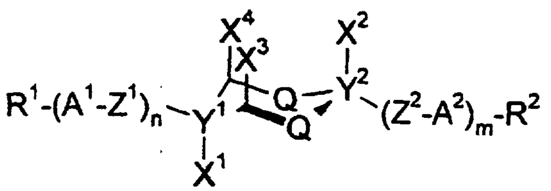

I

worin

$R^1$, $R^2$ unabhängig voneinander H, F, einen unsubstituierten, einen mindestens einfach durch Halogen oder CN substituierten Alkylrest mit 1-15 C-Atomen, worin auch eine oder mehrere $CH_2$-Gruppen jeweils unabhängig

voneinander durch -O-, -S-, -CO-, 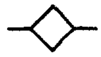, -CO-O-, -O-CO-, -O-CO-O- oder -CH=CH- so ersetzt sein können, dass Heteroatome nicht direkt verbunden sind.

$X^1$, $X^2$, $X^3$, $X^4$ jeweils unabhängig voneinander H oder -C≡C-C≡C-$R^3$, wobei zumindest eine der Gruppen $X^1$, $X^2$, $X^3$ und $X^4$ -C≡C-C≡C-$R^3$ bedeutet,

$R^3$ H, Cl, CN, $SF_5$, $CF_3$, einen unsubstituierten oder einen mindestens einfach durch Halogen substituierten Alkylrest mit 1-15 C-Atomen, worin auch eine oder mehrere $CH_2$-Gruppen durch jeweils unabhängig voneinander -CH=CH- oder -O- so ersetzt sein können, dass -O-Atome nicht direkt verbunden sind,

Q -$CH_2$- oder -O-,

$Y^1$, $Y^2$ unabhängig voneinander C oder Si,

$A^1$, $A^2$ unabhängig voneinander einen unsubstituierten oder durch F oder CN substituierten trans-1,4-Cyclohexylenrest, worin auch eine oder mehrere nicht benachbarte $CH_2$-Gruppen durch -O- und/oder -S- ersetzt sein können, oder

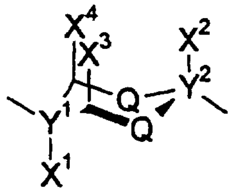

,

$Z^1$, $Z^2$ jeweils unabhängig voneinander -CO-O-, -O-CO-, -$CH_2$O-, -O-, -O$CH_2$-, -$CF_2$O-, -O$CF_2$-, -$CF_2CF_2$-, -CF=CF-, $CH_2CH_2$-, -CH=CH- oder eine Einfachbindung

und

n, m unabhängig voneinander 0, 1, 2 oder 3,

wobei

m+n 1, 2 oder 3

bedeutet.

**2.** Dialkinverbindungen nach Anspruch 1, **dadurch gekennzeichnet, dass** sie bei einer reduzierten Temperatur von 0,9 und einer Wellenlänge von 589 nm einen Wert für die optische Anisotropie $\Delta n$ von < 0,03 aufweisen.

**3.** Dialkinverbindungen nach Anspruch 1 oder 2, **dadurch gekennzeichnet, dass** $Y^1$ und $Y^2$ C bedeuten.

**4.** Dialkinverbindungen nach Anspruch 1, 2 oder 3, **dadurch gekennzeichnet, dass** m und n 0, 1 oder 2 bedeuten.

**5.** Dialkinverbindungen nach einem der Ansprüche 1 bis 4, **dadurch gekennzeichnet, dass** $Z^1$ und $Z^2$ unabhängig voneinander -$CH_2CH_2$-, -COO-, -OOC-, -$CF_2$O-, -O$CF_2$- oder eine Einfachbindung bedeuten.

**6.** Dialkinverbindungen nach einem der Ansprüche 1 bis 5, **dadurch gekennzeichnet, dass** $R^1$ und $R^2$ gleichzeitig geradkettiges Alkyl oder Alkoxy mit 1 bis 10 C-Atomen bedeuten.

**7.** Dialkinverbindungen nach einem der Ansprüche 1 bis 6, **dadurch gekennzeichnet, dass** $X^1$, $X^2$, $X^3$ und/oder $X^4$ -C≡C-C≡C-CH(Alkyl)$_2$, -C≡C-C≡C-C(Alkyl )(Alkyl)$_2$ oder -C≡C-C≡C-C(Alkyl$^*$)$_3$ bedeuten, wobei Alkyl und Alkyl$^{**}$ jeweils unabhängig voneinander $CH_3$, $C_2H_5$ oder $C_3H_7$ bedeuten.

**8.** Dialkinverbindungen nach einem der Ansprüche 1 bis 7, **dadurch gekennzeichnet, dass** $X^3$ und $X^4$ gleichzeitig H bedeuten.

**9.** Verwendung von Verbindungen der Formel I nach einem der Ansprüche 1 bis 8 als Komponenten flüssigkristalliner Medien.

**10.** Flüssigkristallines Medium mit mindestens zwei flüssigkristallinen Komponenten, **dadurch gekennzeichnet, dass** es mindestens eine Verbindung der Formel I enthält.

11. Flüssigkristall-Anzeigeelement, **dadurch gekennzeichnet, dass** es ein flüssigkristallies Medium nach Anspruch 10 enthält.

12. Reflektives oder transflektives Flüssigkristallanzeigeelement, **dadurch gekennzeichnet, dass** es als Dielektrikum ein flüssigkristallines Medium nach Anspruch 10 enthält.

13. Elektrooptisches Anzeigeelement, **dadurch gekennzeichnet, dass** es als Dielektrikum ein flüssigkristallines Medium nach Anspruch 10 enthält.

## Claims

1. Dialkyne compounds of the formula I

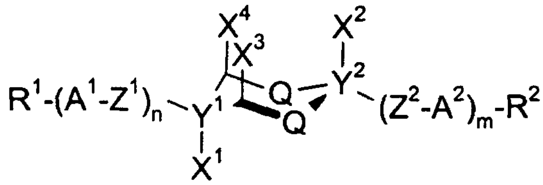

I

in which

$R^1$, $R^2$, independently of one another, denote H, F, an alkyl radical having 1-15 C atoms which is unsubstituted or at least monosubstituted by halogen or CN and in which one or more $CH_2$ groups may each, independently of one another, be replaced by -O-, -S-, -CO-, 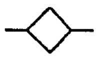, -CO-O-, -O-CO-, -O-CO-O- or -CH=CH- in such a way that heteroatoms are not connected directly,
$X^1$, $X^2$, $X^3$, $X^4$ each, independently of one another, denote H or $-C{\equiv}C-C{\equiv}C-R^3$, where at least one of the groups $X^1$, $X^2$, $X^3$ and $X^4$ denotes $-C{\equiv}C-C{\equiv}C-R^3$,
$R^3$ denotes H, Cl, CN, $SF_5$, $CF_3$, an alkyl radical having 1-15 C atoms which is unsubstituted or at least monosubstituted by halogen and in which one or more $CH_2$ groups may each, independently of one another, be replaced by -CH=CH- or -O- in such a way that -O- atoms are not connected directly,
Q denotes $-CH_2-$ or -O-,
$Y^1$, $Y^2$, independently of one another, denote C or Si,
$A^1$, $A^2$, independently of one another, denote a trans-1,4-cyclohexylene radical which is unsubstituted or substituted by F or CN and in which one or more non-adjacent $CH_2$ groups may be replaced by -O- and/or -S-, or

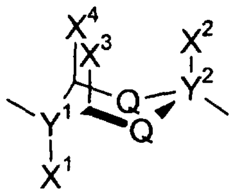

,

$Z^1$, $Z^2$ each, independently of one another, denote -CO-O-, -O-CO-, $-CH_2O-$, -O-, $-OCH_2-$, $-CF_2O-$, $-OCF_2-$, $-CF_2CF_2-$, -CF=CF-, $-CH_2CH_2-$, -CH=CH- or a single bond
and
n, m, independently of one another, denote 0, 1, 2 or 3,
where
m + n denotes 1, 2 or 3.

2. Dialkyne compounds according to Claim 1, **characterised in that** they have an optical anisotropy value Δn of < 0.03 at a reduced temperature of 0.9 and a wavelength of 589 nm.

3. Dialkyne compounds according to Claim 1 or 2, **characterised in that** $Y^1$ and $Y^2$ denote C.

4. Dialkyne compounds according to Claim 1, 2 or 3, **characterised in that** m and n denote 0, 1 or 2.

5. Dialkyne compounds according to one of Claims 1 to 4, **characterised in that** $Z^1$ and $Z^2$, independently of one another, denote $-CH_2CH_2-$, -COO-, -OOC-, $-CF_2O-$, $-OCF_2-$ or a single bond.

6. Dialkyne compounds according to one of Claims 1 to 5, **characterised in that** $R^1$ and $R^2$ simultaneously denote straight-chain alkyl or alkoxy having 1 to 10 C atoms.

7. Dialkyne compounds according to one of Claims 1 to 6, **characterised in that** $X^1$, $X^2$, $X^3$ and/or $X^4$ denote -C≡C-C≡C-CH(alkyl$^*$)$_2$, -C≡C-C≡C-C(alkyl$^*$)(atkyl$^{**}$)$_2$ or -C≡C-C≡C-C(alkyl$^*$)$_3$, where alkyl$^*$ and alkyl each, independently of one another, denote $CH_3$, $C_2H_5$ or $C_3H_7$.

8. Dialkyne compounds according to one of Claims 1 to 7, **characterised in that** $X^3$ and $X^4$ simultaneously denote H.

9. Use of compounds of the formula I according to one of Claims 1 to 8 as components of liquid-crystalline media.

10. Liquid-crystalline medium having at least two liquid-crystalline components, **characterised in that** it comprises at least one compound of the formula I.

11. Liquid-crystal display element, **characterised in that** it contains a liquid-crystalline medium according to Claim 10.

12. Reflective or transflective liquid-crystal display element, **characterised in that** it contains, as dielectric, a liquid-crystalline medium according to Claim 10.

13. Electro-optical display element, **characterised in that** it contains, as dielectric, a liquid-crystalline medium according to Claim 10.

## Revendications

1. Composés de dialkyne de la formule I

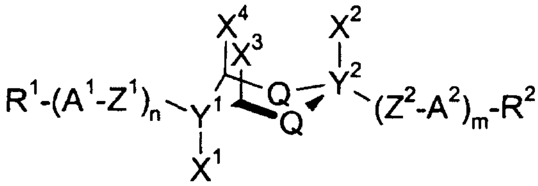

I

dans laquelle

$R^1$, $R^2$, indépendamment l'un de l'autre, représentent H, F, un radical alkyle ayant 1-15 atomes de C qui est non substitué ou au moins monosubstitué par halogène ou CN et dans lequel un ou plusieurs groupes $CH_2$ peuvent chacun, indépendamment les uns des autres, être remplacés par -O-, -S-, -CO-, 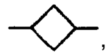, -CO-O-, -O-CO-, -O-CO-O- ou -CH=CH- de telle sorte que des hétéroatomes ne soient pas connectés directement,
$X^1$, $X^2$, $X^3$, $X^4$ indépendamment les uns des autres, représentent chacun H ou -C≡C-C≡C-$R^3$, où au moins l'un des groupes $X^1$, $X^2$, $X^3$ et $X^4$ représente -C≡C-C≡C-$R^3$,
$R^3$ représente H, Cl, CN, $SF_5$, $CF_3$, un radical alkyle ayant 1-15 atomes de C qui est non substitué ou au moins monosubstitué par halogène et dans lequel un ou plusieurs groupes $CH_2$ peuvent chacun, indépendamment les uns des autres, être remplacés par -CH=CH- ou -O- de telle sorte que des atomes de O ne soient pas connectés directement,
Q représente $-CH_2-$ ou -O-,
$Y^1$, $Y^2$, indépendamment l'un de l'autre, représentent C ou Si,

$A^1$, $A^2$, indépendamment l'un de l'autre, représentent un radical trans-1,4-cyclohexylène qui est non substitué ou substitué par F ou CN et dans lequel un ou plusieurs groupes $CH_2$ non adjacents peuvent être remplacés par -O- et/ou -S-, ou

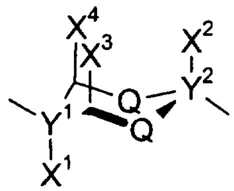

,

$Z^1$, $Z^2$ indépendamment l'un de l'autre, représentent chacun -CO-O-, -O-CO-, $-CH_2O-$, -O-, $-OCH_2-$, $-CF_2O-$, $-OCF_2-$, $-CF_2CF_2-$, -CF=CF-, $-CH_2CH_2-$, -CH=CH- ou une liaison simple
et
n, m, indépendamment l'un de l'autre, représentent 0, 1, 2 ou 3,
dans laquelle
m + n représente 1, 2 ou 3.

2. Composés de dialkyne selon la revendication 1, **caractérisés en ce qu'**ils ont une valeur d'anisotropie optique $\Delta n$ < 0,03 à une température réduite de 0,9 et une longueur d'onde de 589 nm.

3. Composés de dialkyne selon la revendication 1 ou 2, **caractérisés en ce que** $Y^1$ et $Y^2$ représentent C.

4. Composés de dialkyne selon la revendication 1, 2 ou 3, **caractérisés en ce que** m et n représentent 0, 1 ou 2.

5. Composés de dialkyne selon l'une des revendications 1 à 4, **caractérisés en ce que** $Z^1$ et $Z^2$, indépendamment l'un de l'autre, représentent $-CH_2CH_2-$, -COO-, -OOC-, $-CF_2O-$ $-OCF_2-$ ou une liaison simple.

6. Composés de dialkyne selon l'une des revendications 1 à 5, **caractérisés en ce que** $R^1$ et $R^2$ représentent simultanément alkyle ou alkoxy en chaîne droite ayant 1 à 10 atomes de C.

7. Composés de dialkyne selon l'une des revendications 1 à 6, **caractérisés en ce que** $X^1$, $X^2$, $X^3$ et/ou $X^4$ représentent $-C\equiv C-C\equiv C-CH(alkyle^*)_2$, $-C\equiv C-C\equiv C-C(alkyle^*)(alkyle^{**})_2$ ou $-C\equiv -C-C\equiv C-C(alkyle^*)_3$, où alkyle* et alkyle** chacun, indépendamment l'un de l'autre, représentent $CH_3$, $C_2H_5$ ou $C_3H_7$.

8. Composés de dialkyne selon l'une des revendications 1 à 7, **caractérisés en ce que** $X^3$ et $X^4$ représentent simultanément H.

9. Utilisation de composés de la formule I selon l'une des revendications 1 à 8 en tant que composants de milieux de cristal liquide.

10. Milieu de cristal liquide ayant au moins deux composants de cristal liquide, **caractérisé en ce qu'**il comprend au moins un composé de la formule I.

11. Elément d'affichage à cristal liquide, **caractérisé en ce qu'**il contient un milieu de cristal liquide selon la revendication 10.

12. Elément d'affichage à cristal liquide par réflexion ou transflexion, **caractérisé en ce qu'**il contient, en tant que diélectrique, un milieu de cristal liquide selon la revendication 10.

13. Elément d'affichage électro-optique, **caractérisé en ce qu'**il contient, en tant que diélectrique, un milieu de cristal liquide selon la revendication 10.